# EUROPEAN PATENT APPLICATION

(11) **EP 4 700 669 A1**
(43) Date of publication of application: **25.02.2026**
(21) Application number: 24792758.5
(22) Date of filing: 19.04.2024
(51) Int. Cl.: G06Q 10/04, G16H 20/10

(54) **COMPUTER PROGRAM, INFORMATION PROCESSING METHOD, INFORMATION PROCESSING DEVICE, AND METHOD FOR GENERATING MODEL**

(30) Priority: 20.04.2023 JP 2023069449; 20.04.2023 JP 2023069450
(71) Applicant: Terumo Kabushiki Kaisha, Tokyo 151-0072 (JP); Juntendo Educational Foundation, Tokyo 113-8421 (JP)
(72) Inventor: KONDO, Akihide, Tokyo 113-8421 (JP); SHIMIZU, Yuzaburo, Tokyo 113-8421 (JP); GOMA, Masaki, Ashigarakami-gun, Kanagawa 259-0151 (JP); HABU, Yoshiyuki, Ashigarakami-gun, Kanagawa 259-0151 (JP); YAMADA, Kazuhiro, Ashigarakami-gun, Kanagawa 259-0151 (JP); ICHIKAWA, Ryo, Ashigarakami-gun, Kanagawa 259-0151 (JP)
(74) Representative: Prüfer & Partner mbB Patentanwälte · Rechtsanwälte
(86) International application number: PCT/JP2024/015561
(87) International publication number: WO 2024/219486

(57) **Abstract**

A computer program causes a computer to perform a process including: acquiring information regarding urine outputs measured from the same patient at a plurality of time points within a predetermined period; inputting the acquired information regarding the urine outputs measured at the plurality of time points to a prediction model that has learned to output information regarding a predicted urine output a predetermined elapsed time ahead when the information regarding the urine outputs measured at the plurality of time points is input; acquiring the information regarding the predicted urine output the predetermined elapsed time ahead, from the prediction model; and outputting the acquired information regarding the predicted urine output.

## Description

### Technical Field

The present invention relates to a computer program, an information processing method, an information processing device, and a model generation method.

### Background Art

A physical condition estimating system capable of estimating physical conditions of a user is known (for example, Patent Literature 1). The physical condition estimating system disclosed in Patent Literature 1 estimates physical conditions, using a prediction database related to prediction of transition of vital information.

### Citation List

### Patent Literature

Patent Literature 1: JP 2022-34853 A

### Summary of Invention

### Technical Problem

However, in the physical condition estimating system of Patent Literature 1, the point of outputting a predicted future urine output on the basis of information regarding a plurality of urine outputs measured from the same patient within a predetermined period is not taken into consideration.

An objective of the present disclosure is to provide a computer program and the like that output a predicted future urine output, on the basis of information regarding a plurality of urine outputs measured from the same patient within a predetermined period.

### Solution to Problem

A computer program according to the present mode causes a computer to perform a process including: acquiring information regarding urine outputs measured from the same patient at a plurality of time points within a predetermined period; inputting the acquired information regarding the urine outputs measured at the plurality of time points to a prediction model that has learned to output information regarding a predicted urine output a predetermined elapsed time ahead when the information regarding the urine outputs measured at the plurality of time points is input; acquiring the information regarding the predicted urine output the predetermined elapsed time ahead, from the prediction model; and outputting the acquired information regarding the predicted urine output.

An information processing method according to the present mode causes a computer to perform a process including: acquiring information regarding urine outputs measured from the same patient at a plurality of time points within a predetermined period; inputting the acquired information regarding the urine outputs measured at the plurality of time points to a prediction model that has learned to output information regarding a predicted urine output a predetermined elapsed time ahead when the information regarding the urine outputs measured at the plurality of time points is input; acquiring the information regarding the predicted urine output the predetermined elapsed time ahead, from the prediction model; and outputting the acquired information regarding the predicted urine output.

An information processing device according to the present mode includes: an acquisition unit that acquires information regarding urine outputs measured from the same patient at a plurality of time points within a predetermined period; an input unit that inputs the acquired information regarding the urine outputs measured at the plurality of time points to a prediction model that has learned to output information regarding a predicted urine output a predetermined elapsed time ahead when the information regarding the urine outputs measured at the plurality of time points is input; and an output unit that outputs the information regarding the predicted urine output the predetermined elapsed time ahead, the information regarding the predicted urine output being acquired from the prediction model.

### Advantageous Effects of Invention

According to the present disclosure, it is possible to provide a computer program and the like that output a predicted future urine output, on the basis of information regarding a plurality of urine outputs measured from the same patient within a predetermined period.

### Brief Description of Drawings

Fig. 1 is a schematic diagram illustrating an outline of an information processing system including an information processing device (a urine output level predicting device) according to a first embodiment.
Fig. 2 is a block diagram illustrating an example configuration of the information processing device.
Fig. 3 is a block diagram illustrating an example configuration of each functional unit formed by a control unit of the information processing device.
Fig. 4 is an explanatory diagram illustrating an example of a prediction model.
Fig. 5 is a flowchart illustrating an example of processing procedures (during learning) to be carried out by the control unit of the information processing device.
Fig. 6 is an explanatory diagram illustrating an example of a urine output level table.
Fig. 7 is an explanatory diagram illustrating an example of a urine discharge type table.
Fig. 8 is an explanatory diagram illustrating an example of an intervention means table.
Fig. 9 is a flowchart illustrating an example of processing procedures (during an operation/urine output level prediction) to be carried out by the control unit of the information processing device.
Fig. 10 is an explanatory diagram illustrating an example of transitions of urine output levels.
Fig. 11 is an explanatory diagram illustrating a display example (display screen) of information regarding a urine output level and the like.
Fig. 12 is a schematic diagram illustrating an outline of an information processing system including an information processing device (a pump controlling device) according to a second embodiment.
Fig. 13 is a block diagram illustrating an example configuration of the information processing device.
Fig. 14 is an explanatory diagram illustrating a flow of various kinds of data for a control unit of the information processing device.
Fig. 15 is a block diagram illustrating an example configuration of each functional unit formed by the control unit of the information processing device.
Fig. 16 is an explanatory diagram illustrating an example of transition of actual measured urine output values and the like.
Fig. 17 is an explanatory diagram illustrating an example of a dosage amount table.
Fig. 18 is a flowchart illustrating an example of processing procedures (actual measured urine output values in the past and till the present time) to be carried out by the control unit of the information processing device.
Fig. 19 is an explanatory diagram illustrating an example of an intervention means table according to a third embodiment.
Fig. 20 is a flowchart illustrating an example of processing procedures (actual measured urine output values and a predicted urine output value) to be carried out by a control unit of an information processing device.

### Description of Embodiments

The present invention will be specifically described on the basis of the drawings illustrating embodiments thereof. An information processing device 1 and the like according to embodiments of the present disclosure will be described below, with reference to the drawings. Note that the present invention is not limited by these embodiments but is disclosed in the claims, and is intended to include all changes within the meaning and scope equivalent to the claims.

### First Embodiment

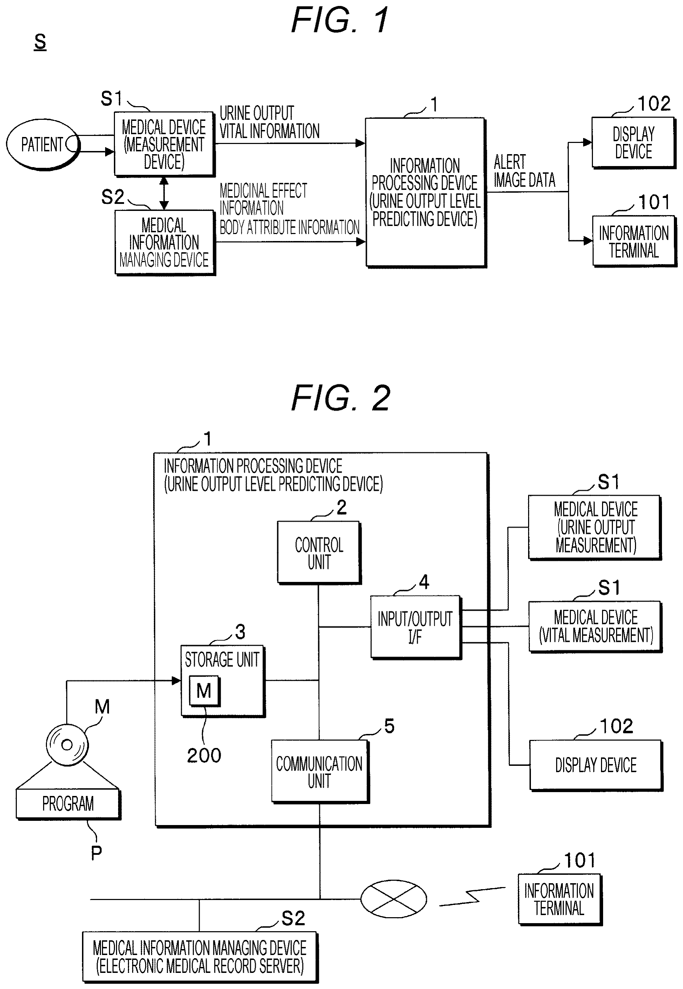

In the description below, the present disclosure is specifically described, with reference to the drawings illustrating an embodiment. Fig. 1 is a schematic diagram illustrating an outline of an information processing system S including an information processing device 1 (a urine output level predicting device) according to a first embodiment. Fig. 2 is a block diagram illustrating an example configuration of the information processing device 1. The information processing system S (a urine output level predicting system) includes the information processing device 1 (urine output level predicting device) as a principal device, and one or more medical devices S1 are communicably connected to the information processing device 1. The medical devices S1 include a medical device S1 for urine output measurement, and a medical device S1 for measurement of information about vital signs (hereinafter also referred to as vital information), for example.

The information processing device 1 periodically acquires, from these medical devices S1, a urine output value of the patient and vital information (biological data) such as a heart rate. The information processing device 1 is further communicably connected to a medical information managing device S2 such as an electronic medical record system that is operated and managed by a medical institution, and acquires physical attribute information such as the patient's weight from the medical information managing device S2. The information processing device 1 may further acquire, from the medical information managing device S2, the type, the dosage amount, and the time point of administration of a medicine administered to the patient, or medicinal effect information. The medicine includes an antidiuretic hormone agent for central diabetes insipidus, for example.

On the basis of various kinds of information acquired from the medical devices S1 and the medical information managing device S2, the information processing device 1 derives information regarding a predicted urine output a predetermined elapsed time ahead, using a prediction model 200 stored in a storage unit 3 of the information processing device 1. On the basis of the derived (predicted) information regarding the predicted urine output (the urine output level of the predicted urine output) and information regarding the actual measured urine outputs from the past up to the present (the urine output levels of the actual measured urine outputs), the information processing device 1 derives the change/transition of these urine output levels from the past to the future, and identifies the urine discharge type on the basis of the change/transition. As the information processing device 1 outputs an alert and an intervention means depending on the urine discharge type identified by taking the predicted future urine output into account, it is possible to predict in advance which pattern of polyuria (urine discharge type) will occur and when the polyuria will occur in the course of time for each patient, and present, to a medical expert such as a medical doctor, information for supporting early consideration of introduction of an intervention means suited to the pattern of polyuria (urine discharge type).

The information processing device 1 is a computer capable of various kinds of information processing and transmission/reception of information, and is a server device, a personal computer, or the like, for example. The server device includes not only a single server device, but also a cloud server device formed with a plurality of computers, or a virtual server device. In a case where the information processing device 1 is formed with a cloud server device, for example, like the medical devices S1, the information processing device 1 is not necessarily installed in medical facilities where the patient is present, and may be communicably connected to the medical devices S1 via an external network such as the Internet. The information processing device 1 includes a control unit 2, a communication unit 5, a storage unit 3, and an input/output I/F 4. The information processing device 1 may be included in a medical device S1 such as a urine output meter, or in a syringe pump or an infusion pump.

The control unit 2 includes an arithmetic processing device having a timing function, such as one or a plurality of central processing units (CPUs), micro-processing units (MPUs), graphics processing units (GPUs), or the like, and performs various kinds of information processing, control processes, and the like related to the information processing device 1 by reading and executing a program P (program product) stored in the storage unit 3.

The storage unit 3 includes a volatile storage area such as a static random access memory (SRAM), a dynamic random access memory (DRAM), or a flash memory, and a nonvolatile storage area such as an EEPROM or a hard disk. The program P (program product) and the data to be referred to during processing are stored into the storage unit 3 beforehand. The program P (program product) stored in the storage unit 3 may be a program P (program product) read from a recording medium M that can be read by the information processing device 1. Alternatively, the program P (program product) may be a program that is downloaded from an external computer (not shown) connected to a communication network (not shown), and is stored into the storage unit 3.

The storage unit 3 stores a urine output level table, a urine discharge type table, and an intervention means table, which will be described later. The storage unit 3 further stores an entity file that constitutes the prediction model 200. The entity file may be formed as part of the program P (program product).

The communication unit 5 is a communication module or a communication interface for communicating, in a wired or wireless manner, with the medical information managing device S2 or an information terminal 101 such as a smartphone held by medical personnel, and is a wired communication module such as an Ethernet (registered trademark) connector, a narrow-area wireless communication module such as WiFi (registered trademark) or Bluetooth (registered trademark), or a wide-area wireless communication module such as 4G or 5G, for example. The control unit 2 communicates with the medical information managing device S2 or the information terminal 101 via the communication unit 5, or, for example, via an external network such as a local network in the medical institution or the Internet.

The input/output I/F 4 is a communication interface conforming to a communications standard such as RS232C or USB, for example. An input device such as a keyboard or a display device 102 such as a liquid crystal display is connected to the input/output I/F 4. Further, the medical devices S1 may be connected to the input/output I/F 4.

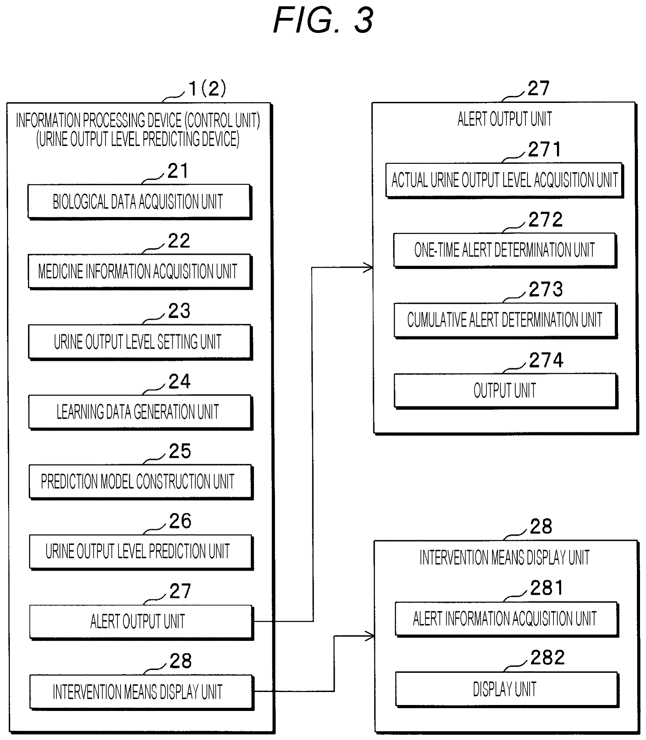

Fig. 3 is a block diagram illustrating an example configuration of each functional unit formed by the control unit 2 of the information processing device 1. The control unit 2 of the information processing device 1 functions as functional units that perform various kinds of software processes by executing the program P stored in the storage unit 3. The functional units include a biological data acquisition unit 21, a medication information acquisition unit 22, a urine output level setting unit 23, a learning data generation unit 24, a prediction model construction unit 25, a urine output level prediction unit 26, an alert output unit 27, and an intervention means display unit 28, for example.

The biological data acquisition unit 21 periodically or regularly acquires the value of a urine output of a patient from a medical device S1 installed for the patient. The biological data acquisition unit 21 may further periodically or regularly acquire a value of urine specific gravity of the patient. The acquired values of the urine output and the urine specific gravity may define (constitute) information regarding the urine output. The biological data acquisition unit 21 may further acquire, from a medical device S1, information (biological data) regarding vital signs such as a heart rate and a blood pressure of the patient. The biological data acquisition unit 21 may acquire the value of a urine output and vital information (biological data) by receiving an input operation by an operator such as medical personnel from an input device such as a keyboard connected to the input/output I/F 4 of the information processing device 1. Note that "vital signs" in the present application refer to vital signs excluding the urine output. Also, the "information regarding vital signs" includes not only measured values of vital signs, but also values calculated from the measured values of vital signs such as a rate of change in blood pressure value within a predetermined time.

The biological data acquisition unit 21 may further acquire body fluid volume information about the patient by receiving an input from a medical device S1 or the medical information managing device S2 installed for the patient, or from a medical worker or the like. The body fluid volume information may use an index that is a balance between the amount of intake of body fluid (body fluid IN) and the amount of discharge of body fluid (body fluid OUT) in a predetermined time (a cumulative balance in a predetermined time X (the total of the body fluid IN in the X time - the total of the body fluid OUT in the X time)). Note that the amount of intake of body fluid (body fluid IN) may be calculated as a total value of the amount of infusion, the amount of drinking water, the amount of medicinal solution, and the like, for example. The amount of discharge of body fluid (body fluid OUT) may be calculated as a total value of the urine output, the stool output, the amount of drainage, the amount of insensible water loss, and the like, for example. In this case, the control unit 2 (biological data acquisition unit 21) may also function as a body fluid I/O acquisition unit. Note that the body fluid volume information may be defined (formed) only by information regarding the amount of intake of body fluid (body fluid IN). That is, in the case of a patient with diabetes insipidus, the amount of discharge of body fluid (body fluid OUT) is substantially equal to the urine output, and accordingly, only the amount of intake of body fluid (body fluid IN) may be used as the body fluid volume information. By grasping the body fluid balance of the patient at the present time (current time) in greater detail, it is possible to estimate an optimal amount of infusion that reduces the burden on the patient and reduces the risk of dehydration. By outputting the estimate of the amount of infusion, it is possible to present useful information to the medical personnel.

The biological data acquisition unit 21 may further acquire a serum sodium level of the patient from a medical device S1 installed for the patient or by receiving an input from a medical worker or the like. In this case, the control unit 2 (biological data acquisition unit 21) may also function as a test value acquisition unit (serum Na level acquisition unit). For example, in the case of a patient with a small body weight such as an infant, even when the amount of urine discharge is small over several hours, body fluid is positively balanced and retained by infusion in preparation for polyuria. At such times, the serum sodium level (serum Na) shows a declining trend. From this declining tendency of the serum sodium level (serum Na), it may be determined that the subject is in a trend of body fluid retention, and it may be determined that there is a possibility that polyuria will occur thereafter. This makes it easier to grasp the trend over time than from the body fluid volume information (body fluid I/O). The risk of hyponatremia can be evaluated by the absolute value. Even for a patient with diabetes insipidus, it can be presumed that polyuria will occur when the body fluid is in a positive balance, and the amount of medicine for the antidiuretic hormone can be appropriately determined (as to whether to reduce it or whether not to administer it at all). In preparation for polyuria in the future, it is possible to estimate an optimum amount of infusion with a smaller burden on the patient and a lower risk of dehydration, and it is possible to present useful information to the medical personnel by outputting the estimated amount of infusion.

The medication information acquisition unit 22 acquires, from the medical information managing device S2, for example, information regarding the type, amount, and time of administration of the medicine most recently administered to the patient, and derives (acquires) medicinal effect information at the present time by referring to the residual effect characteristics of the medicine, depending on the time elapsed since the time of administration till the present time.

The urine output level setting unit 23 receives an operation by the operator of the information processing device 1 from an input device such as a keyboard connected to the input/output I/F 4 of the information processing device 1, for example, and updates the urine output level table on the basis of the received operation, to change the set value of the urine output level or the like.

The learning data generation unit 24 acquires a data set (information regarding a plurality of urine outputs sorted on a time-series basis, medicinal effect information, vital information, body fluid volume information, and serum sodium level) about each patient from an electronic medical record system, a medical DB system, or the like operated and managed by a medical institution such as a hospital, for example, and generates learning data (training data) for causing the prediction model 200 to learn using the data set. In the data set, a plurality of pieces of data sorted on a time-series basis is not necessarily information regarding urine outputs (urine output values or urine output levels). The data set may include a plurality of pieces of medicinal effect information sorted on a time-series basis, a plurality of pieces of vital information sorted on a time-series basis, a plurality of pieces of body fluid volume information sorted on a time-series basis, and a plurality of serum sodium levels sorted on a time-series basis. In this case, the time interval (the unit time of sampling: measurement unit time) defined by the time series in the information regarding urine outputs may be different from the time interval (the unit time of sampling: measurement unit time) defined by the time series in the medicinal effect information, the vital information, the body fluid volume information, or the serum sodium level. In a case where the measurement unit time defined by the time series in the information regarding urine outputs is one hour, for example, the measurement unit time in the body fluid volume information or the serum sodium level may be set to three hours, for example, and the measurement unit time in the information regarding urine outputs may be set to a shorter time than the measurement unit time in the other information.

The medicinal effect information, the vital information, the body fluid volume information, and the serum sodium level are not limited to those in a case with a plurality of pieces of data sorted on a time-series basis, and any of the information may be only data of the present time (current time), which means that the data set is intended to include all combinations thereof. The learning data generation unit 24 does not necessarily use a data set including all of information regarding a plurality of urine outputs sorted on a time-series basis, medicinal effect information, vital information, body fluid volume information, and serum sodium level. The learning data generation unit 24 may use a data set including any one or more pieces of medicinal effect information, vital information, body fluid volume information, and serum sodium level, in addition to information regarding a plurality of urine outputs sorted on a time-series basis. That is, the data set is intended to include, as main data, information regarding a plurality of urine outputs sorted on a time-series basis, and is formed with all combinations including any one or more pieces of medicinal effect information, vital information, body fluid volume information, and serum sodium level.

The prediction model construction unit 25 generates (constructs) the prediction model 200 by applying the learning data (training data) generated by the learning data generation unit 24 to a neural network such as a DNN, and causing the neural network to learn.

The urine output level prediction unit 26 derives information regarding a predicted urine output (the urine output level of a predicted urine output), using the prediction model 200 generated (constructed) by the prediction model construction unit 25.

The alert output unit 27 includes an actual urine output level acquisition unit 271, a one-time alert determination unit 272, a cumulative alert determination unit 273, and an output unit 274, for example. The actual urine output level acquisition unit 271 acquires a plurality of urine outputs (actual measured urine outputs) from the past up to the present acquired by the biological data acquisition unit 21, and acquires (identifies) the urine output levels of these actual measured urine outputs.

The one-time alert determination unit 272 determines whether the information regarding a predicted urine output derived (predicted) by the urine output level prediction unit 26 (the urine output level of the predicted urine output) matches an excessive urine output defined in the urine discharge type table described later, and outputs the result of the determination. If the hourly prediction result indicates an excessive urine output (red) according to the one-time alert determination made by the one-time alert determination unit 272, for example, an alert can be issued.

The cumulative alert determination unit 273 determines whether the urine output levels from the past to the future acquired and predicted by the actual urine output level acquisition unit 271 and the urine output level prediction unit 26 match a mode in which the urine outputs are continued (accumulated) at the intermediate urine output defined in the urine discharge type table, and outputs the result of the determination. Depending on the cumulative alert determination by the cumulative alert determination unit 273, an alert is output when a state in which the actual urine output level indicates the intermediate urine output (yellow) has continued for two hours and the intermediate urine output (yellow) is predicted (the urine output level of the predicted urine output) at the third hour, for example, without determination only by the hourly prediction result.

The output unit 274 outputs the determination results from the one-time alert determination unit 272 and the cumulative alert determination unit 273 to the display device 102 or the information terminal 101, for example.

The intervention means display unit 28 includes an alert information acquisition unit 281 and a display unit 282, for example. The alert information acquisition unit 281 acquires, from the alert output unit 27 (output unit 274), the result of the determination made by the one-time alert determination unit 272 or the cumulative alert determination unit 273. On the basis of the acquired determination result and the like (including the urine discharge type), the alert information acquisition unit 281 derives an intervention means corresponding to the determination result (urine discharge type), by referring to the intervention means table, for example.

The display unit 282 acquires the intervention means from the alert information acquisition unit 281, and outputs the intervention means to the display device 102 or the information terminal 101. The display unit 282 may output an alert, together with the intervention means, to the display device 102 or the information terminal 101.

The development to various functional units in the present embodiment is an example, and the present invention is not limited to this. The control unit 2 of the information processing device 1 may be developed (function) as a functional unit in a form in which the above functional units are further distributed or are integrated, depending on the module configuration according to the program to be executed.

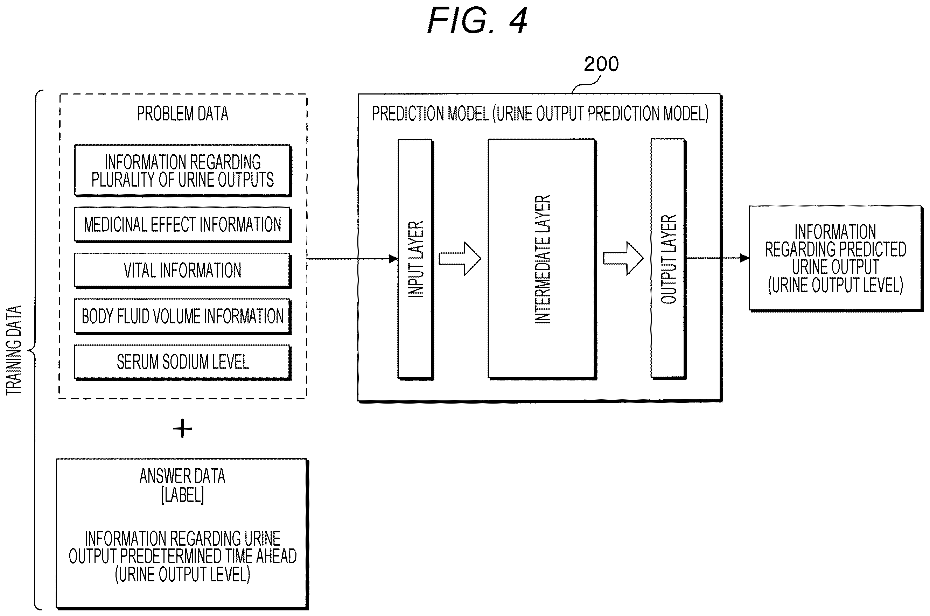

Fig. 4 is an explanatory diagram illustrating an example of the prediction model 200. The control unit 2 of the information processing device 1 causes a neural network to learn using the training data for the prediction model 200, and generates the prediction model 200 that receives information regarding a plurality of urine outputs, medicinal effect information, and vital information as inputs, and outputs information regarding a predicted urine output at a predetermined elapsed time ahead. The predicted urine output information to be output by the prediction model 200 may be a urine output level defined by the urine output value of the predicted urine output.

The neural network (prediction model 200) that has learned using the training data is assumed to be used as a program module that is part of artificial intelligence software. The prediction model 200 is used in the information processing device 1 including the control unit 2 (a CPU and the like) and the storage unit 3 as described above, and is executed by the information processing device 1 having arithmetic processing capability as above, to form a neural network system. That is, the control unit 2 of the information processing device 1 operates to perform an arithmetic operation of extracting a feature amount such as information regarding a plurality of urine outputs input to an input layer in accordance with an instruction from the prediction model 200 stored in the storage unit 3, and output information regarding a predicted urine output from an output layer.

The prediction model 200 is formed with a deep neural network (DNN), for example, and includes an input layer that receives inputs of information regarding a plurality of urine outputs, medicinal effect information, and vital information, an intermediate layer that extracts a feature amount such as the information regarding a plurality of urine outputs, and an output layer that outputs information regarding a predicted urine output. The input layer may receive body fluid volume information and information regarding serum sodium level, in addition to the information regarding a plurality of urine outputs, the medicinal effect information, and the vital information. The input layer has a plurality of neurons that receive inputs of the information regarding a plurality of urine outputs, the medicinal effect information, the vital information, the body fluid volume information, the serum sodium level, and the like, and passes the input values on to the intermediate layer. The intermediate layer is defined using an activating function such as a ReLu function or a sigmoid function, includes a plurality of neurons that extract a feature amount of each input value, and passes the extracted feature amounts on to the output layer. Parameters such as a weighting coefficient and a bias value of the activating function are optimized using the error back-propagation method. The output layer is formed with a fully connected layer, for example, and outputs the information regarding a predicted urine output (the urine output level of the predicted urine output), on the basis of the feature amount output from the intermediate layer. The output layer may output the value of a predicted urine output (predicted urine output value) as the information regarding a predicted urine output.

In the present embodiment, the prediction model 200 is a DNN, but is not limited to this. The prediction model 200 may be a learning model constructed by some other learning algorithm such as a neural network other than a DNN, a recurrent neural network (RNN), a long-short term model (LSTM), a CNN, a support vector machine (SVM), a Bayesian network, a linear regression, a multiple regression, a regression tree, a classification tree, a light gradient boosting machine (LightGBM), a random forest, or an ensemble. Note that a data set of problem data and answer data included in the training data for training the prediction model 200, and the data set of the input data and the output data used when the prediction model 200 is used are synonymous, and are naturally applied to other data sets as long as they are defined in any of the data sets.

For example, in a case where the prediction model 200 is generated using a regression model, both the input and the output to the prediction model 200 may use a urine output value. In this case, when a plurality of actual measured urine output values sorted on a time-series basis is input, the prediction model 200 generated with a regression model outputs (estimates) a predicted urine output value.

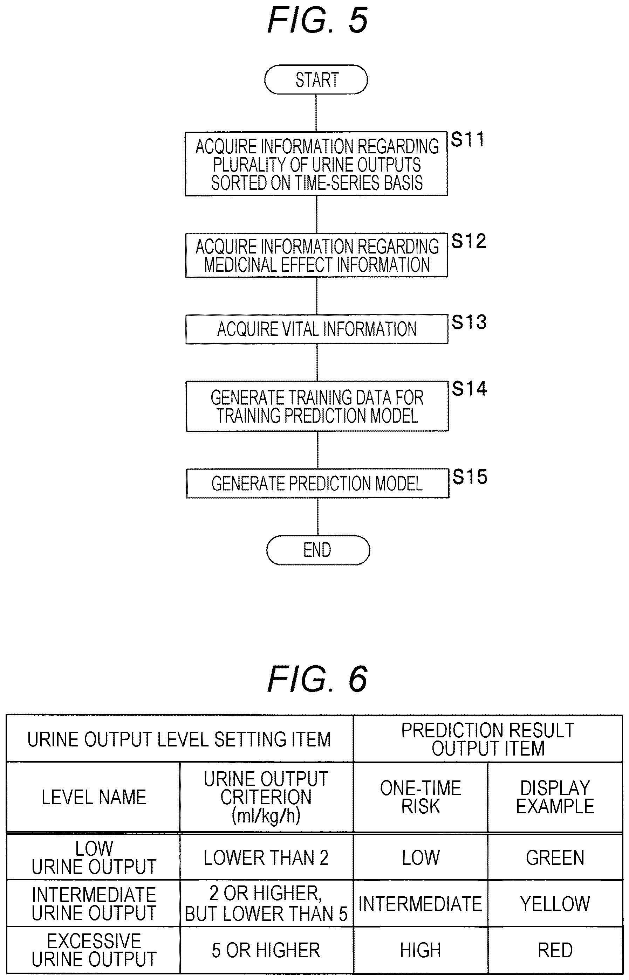

Fig. 5 is a flowchart illustrating an example of processing procedures (during learning) to be carried out by the control unit 2 of the information processing device 1. The control unit 2 of the information processing device 1 accepts an operator's operation using a keyboard or the like connected to the input/output I/F 4, for example, and performs the following process on the basis of the accepted operation.

The control unit 2 of the information processing device 1 acquires information regarding a plurality of urine outputs sorted on a time-series basis (S11). The control unit 2 of the information processing device 1 acquires information regarding medicinal effect information (S12). The control unit 2 of the information processing device 1 acquires vital information (S13). The control unit 2 of the information processing device 1 acquires the information regarding a plurality of urine outputs of the same patient sorted on a time-series basis, the information on the medicinal effect information, and the vital information. The control unit 2 of the information processing device 1 may acquire these pieces of information as a data set corresponding to the patient. The control unit 2 of the information processing device 1 acquires a plurality of such data sets of the respective patients, or a data set of each patient of a plurality of patients. The data set (a plurality of urine output values sorted on a time-series basis, medicinal effect information, and vital information) of each patient is stored in an electronic medical record system, a medical DB system, or the like operated and managed in a medical institution such as a hospital, and the control unit 2 of the information processing device 1 can acquire the data set of each patient among a large number of patients by accessing the electronic medical record system or the like.

The control unit 2 of the information processing device 1 generates training data for training the prediction model 200 (S14). The control unit 2 of the information processing device 1 generates the training data for training the prediction model 200, using the acquired data set of each patient. The control unit 2 of the information processing device 1 may use, as the answer data, the urine output value at the last measurement time point among a plurality of pieces of acquired information regarding a plurality of urine outputs sorted on a time-series basis, which is a plurality of urine output values sorted on a time-series basis within a measurement unit time, and may use a plurality of urine output values at measurement time points earlier than the last measurement time point as the problem data. The answer data may be presented as a urine output level defined by a urine output value. Further, the plurality of urine output values included in the problem data may be presented as urine output levels defined by the urine output value. Further, the problem data may include statistics (such as a total value, an average, a maximum value, a minimum value, an amount of change, and a rate of change) of the plurality of urine output values. Further, the control unit 2 of the information processing device 1 may incorporate, into the problem data, information (a medicinal effect index indicating the residual effect) regarding the medicinal effect information and the vital information at the same time point as the measurement time point (the time point of measurement of the urine output value last measured among the plurality of urine output values included in the answer data) immediately before the last measurement time point. The control unit 2 of the information processing device 1 can generate large-capacity training data by acquiring the data sets of a large number of patients.

The control unit 2 of the information processing device 1 generates the prediction model 200, using the generated training data (S15). For example, the control unit 2 of the information processing device 1 causes a neural network such as a DNN to learn using training data generated for the DNN, to generate the prediction model 200 that outputs a predicted urine output a predetermined elapsed time ahead (after a measurement unit time), in a case where information regarding a plurality of urine outputs, information regarding medicinal effect information, and vital information are input thereto.

In the present embodiment, the prediction model 200 learns using information regarding a plurality of urine outputs, medicinal effect information, and vital information, but the present invention is not limited to this. The prediction model 200 may perform learning using only the information regarding a plurality of urine outputs, only the information regarding a plurality of urine outputs and the medicinal effect information, or only the information regarding a plurality of urine outputs and the vital information.

In the storage unit 3 of the information processing device 1, various kinds of setting information to be referred to when the control unit 2 of the information processing device 1 performs various kinds of arithmetic processing are stored in a table format, for example. The various kinds of setting information (tables) stored in a table format include a urine output level table, a urine discharge type table, and an intervention means table, for example.

Fig. 6 is an explanatory diagram illustrating an example of the urine output level table. The management items (fields) in the urine output level table include level name, urine output criterion, one-time risk, and display example, for example. In the level name management item, level names defining urine output levels, such as low urine output, intermediate urine output, and excessive urine output, are stored, for example. In a urine output criteria management item, the urine output criterion (ml/kg/h) corresponding to the level name stored in the same record is stored, for example. As for the correspondence between the level name and the urine output criterion (ml/kg/h) may be the low urine output in a case where the urine output criterion (ml/kg/h) is less than 2, the intermediate urine output in a case where the urine output criterion (ml/kg/h) is 2 or higher but lower than 5, and the excessive urine output in a case where the urine output criterion (ml/kg/h) is 5 or higher, for example. The urine output level setting item is formed with the level name and the urine output criterion.

In a one-time risk management item, a value (low, medium, high) indicating the degree of the one-time risk corresponding to the level name stored in the same record is stored, for example. In a display example management item, a display color (coloring) of a display object indicating the level name when the level name stored in the same record is displayed on the screen is stored, for example. A prediction result output item is formed with a one-time risk and a display example.

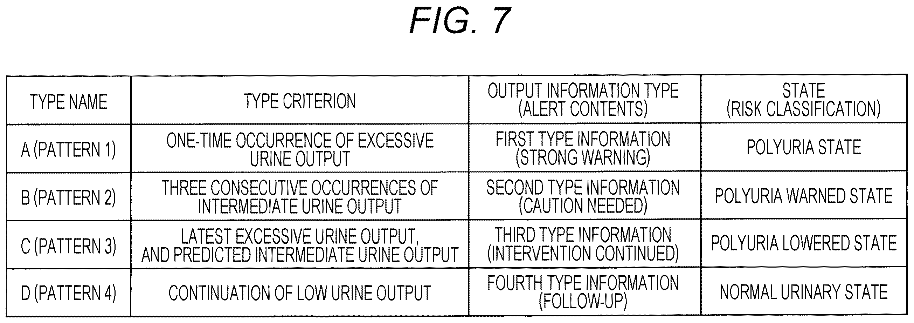

Fig. 7 is an explanatory diagram illustrating an example of the urine discharge type table. The management items (fields) in the urine discharge type table include type name, type criterion, and output information type (alert content), for example. In a type name management item, a type name for dividing or classifying urine discharge types is stored, for example. The type name may include a value indicating the risk in each urine discharge type (degrees of risks: A > B > C > D).

In a type criterion management item, the criterion corresponding to the type name stored in the same record is stored. The criterion (type criterion) is determined on the basis of a transition including the urine output level of a predicted urine output. For example, a case where the urine output level of the predicted urine output is an excessive urine output corresponds to a type criterion (A (pattern 1)) indicating a one-time occurrence of an excessive urine output. A case where the urine output levels of the past and present actual measured urine outputs and the predicted future urine output are intermediate urine outputs corresponds to a type criterion (B (pattern 2)) indicating three consecutive occurrences (cumulation) of an intermediate urine output. A case where the current urine output is an excessive urine output, and the urine output level of the predicted future urine output is an intermediate urine output corresponds to a type criterion (C (pattern 3)) indicating that the symptom is improving. A case where the urine output levels of the past and present actual measured urine outputs and the predicted future urine output are low urine outputs corresponds to a type criterion (D (pattern 4)) indicating that a low urine output has continued. The type criteria defined in this manner are not limited to those in the case of being defined (determined) by the urine output levels at three points in the past, at the present, and in the future, and may be defined (determined) by the urine output levels at two points at the present and in the future. Alternatively, the type criteria may be defined (determined) by the urine output levels at four or more points two or more types ago in the past, at the present, and in the future. The number of the past measurement time points (the number of measurement time points) retroactive in this manner is not limited to that in a case where the number is set in a fixed manner, but may be variably set in accordance with an input or a change operation performed by an operator of the information processing device 1, such as a medical personnel.

In an output information type (alert content) management item, the type or content of the output information (alert) corresponding to the type name stored in the same record is stored. The output information (alert) may be output as a voice from a speaker or the like, for example, or may be output as a character string or the like on a display screen of the information terminal 101 or the display device 102.

Further, the management items (fields) in the urine discharge type table may also include an item indicating states (risk classifications) under the respective type names. For example, a type (A (pattern 1)) having a visible risk of polyuria is in a polyuria state (polyuria type [high]), and a type (B (pattern 2)) having a relatively high risk of polyuria is in a polyuria warned state (polyuria type [intermediate]). A type (C (pattern 3)) having a relatively low risk of polyuria (the risk of polyuria is reducing) is in a polyuria lowering state (polyuria type [low]). A type (D (pattern 4)) having substantially no risk of polyuria is in a normal urinary state (polyuria type [absent]).

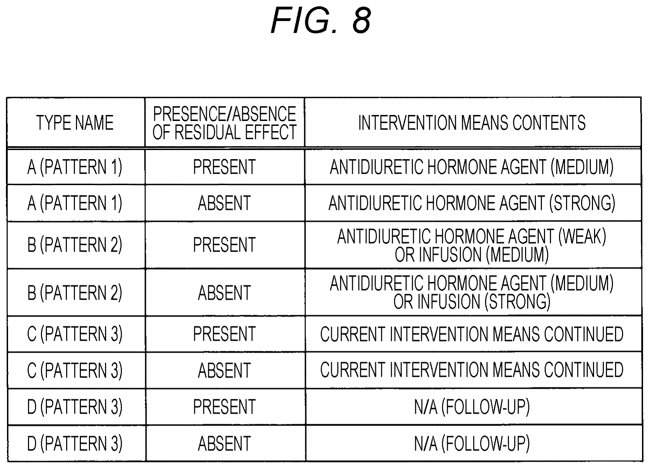

Fig. 8 is an explanatory diagram illustrating an example of the intervention means table. The management items (fields) in the intervention means table include type name, presence or absence of residual effect of medicine, and content of intervention means, for example. In a type name management item, a type name defined in the urine discharge type table is stored. With this arrangement, the intervention means table and the urine discharge type table are normalized by the type name management items. In a management item of the presence or absence of the residual effect of the medicine, a value (presence/absence) indicating the presence or absence of a residual effect of the medicine is stored under the respective type names (A to D, for example) defined by the urine discharge types.

In a management item of the content of an intervention means, the content of the intervention means corresponding to the combinations of the type name and the presence or absence (presence/absence) of a residual effect of the medicine stored in the same record is stored. As the combinations are defined, the control unit 2 of the information processing device 1 can efficiently derive the intervention means corresponding to the combination of the identified urine discharge type and the acquired medicinal effect information.

In the present embodiment, the classification of the urine output levels and the urine discharge types is an example, and the present invention is not limited to this. The urine output levels may be further subdivided and defined depending on the urine output values. The urine discharge types may be further subdivided and defined depending on modes of transitions (changes) of a plurality of urine output levels that are continuous in time series. Further, the value or content of each of the items defined or managed in the urine output level table, the urine discharge type table, and the intervention means table is not necessarily set in a fixed manner, but may be variably set depending on an input or a change operation performed by an operator of the information processing device 1, such as a medical personnel. That is, the control unit 2 of the information processing device 1 may receive an input or a change operation performed by an operator such as a medical personnel, and change or update the urine output level table, the urine discharge type table, or the intervention means table, depending on the received operation content. For example, as for the definition of a urine discharge type or the like, even in a case where there is a difference in the criteria of the definition at each medical institution, it is possible to allow changes in the settings or definitions in each table in this manner, and increase availability of the information processing system S (a urine output level predicting system).

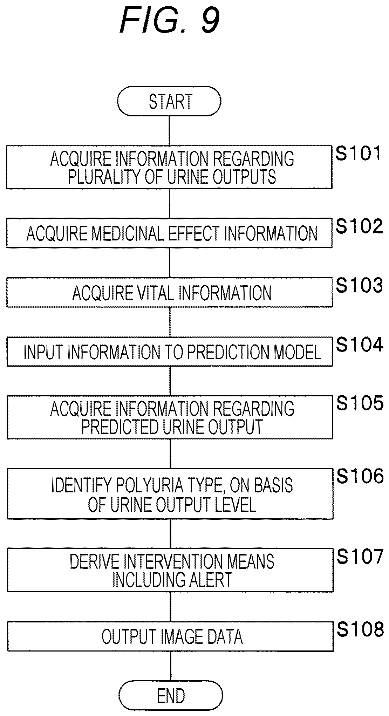

Fig. 9 is a flowchart illustrating an example of processing procedures (during an operation/urine output level prediction) to be carried out by the control unit 2 of the information processing device 1. The control unit 2 of the information processing device 1 performs the following processing, in accordance with an operation by a medical expert such as a medical doctor.

The control unit 2 of the information processing device 1 acquires information regarding a plurality of urine outputs measured from the same patient within a predetermined period (S101). The control unit 2 of the information processing device 1 acquires information regarding a plurality of urine outputs of the same patient within a predetermined period, by periodically or regularly acquiring a urine output value output from a medical device S1 that performs urine output measurement, for example. The medical device S1 for urine output measurement measures the value of a urine output stored in a urine bag of the same patient every measurement unit time such as one hour, for example, and outputs the measured value (the value of the urine output [ml]) to the information processing device 1.

The control unit 2 of the information processing device 1 may acquire the value (the total amount in the urine bag) of the urine output acquired every measurement unit time (one hour or the like, for example), and calculate a value (ml/h) of the urine output in the current measurement unit time by subtracting the value of the urine output acquired last time from the value of the urine output acquired this time. Further, the control unit 2 of the information processing device 1 may acquire physical attribute information including the body weight and the like of the patient from the medical information managing device S2, and acquire, as information regarding the urine output, the value of the urine output to the body weight obtained by dividing the value of the urine output (ml/h) in a measurement unit time by the body weight (kg) of the patient (urine output value to body weight: ml/kg/h). That is, the control unit 2 of the information processing device 1 may perform various kinds of arithmetic processing in the present embodiment, using the urine output value to body weight (ml/kg/h) as an actual measured urine output value. Furthermore, the control unit 2 of the information processing device 1 may acquire, together with the urine output, a urine specific gravity output from the medical device S1 that performs urine output measurement, and acquire a plurality of the acquired urine specific gravity values actually measured and sorted on a time-series basis as the information regarding the urine output. The urine specific gravity values may be measured on the basis of a gravimetric method, a floating balance, a refractometer method, or a test paper method, for example, and be automatically acquired from a sensor device or the like, or may be acquired by receiving an input operation by an operator such as a medical personnel. Further, the amount of change in urine specific gravity in a predetermined time calculated from a plurality of urine specific gravity values actually measured and sorted on a time-series basis may be acquired as the information regarding the urine output.

The control unit 2 of the information processing device 1 acquires medicinal effect information about the medicine administered to the patient (S102). The control unit 2 of the information processing device 1 acquires, from the medical information managing device S2, information regarding the type, amount, and time of administration of the medicine most recently administered to the patient, and derives (acquires) medicinal effect information at the present time by referring to the residual effect characteristics of the medicine, depending on the time elapsed since the time of administration till the present time. In the medicinal effect information, a medicinal effect index at the time of administration is set to 100, for example, and the medicinal effect index may be lowered over the lapse of time.

The control unit 2 of the information processing device 1 acquires information regarding vital signs of the patient (S103). The control unit 2 of the information processing device 1 acquires information regarding vital signs output from a medical device S1 that measures information (biological data) regarding vital signs such as heart rate and blood pressure, for example. The control unit 2 of the information processing device 1 may acquire various kinds of data such as the value of a urine output, the medicinal effect information, and information (biological data) regarding vital signs, by receiving an input operation by an operator such as a medical personnel from an input device such as a keyboard connected to the input/output I/F 4. The control unit 2 of the information processing device 1 may further acquire body fluid volume information or serum sodium level, or both pieces of information. In this case, the body fluid volume information may include only the amount of intake of body fluid (body fluid IN), or may include the amount of intake of body fluid (body fluid IN) and the amount of discharge of body fluid (body fluid OUT). The medicinal effect information, the vital information, the body fluid volume information, and the serum sodium level may be data at the present time, or may be formed with a plurality of pieces of data sorted on a time-series basis. The control unit 2 of the information processing device 1 may acquire any combination of the medicinal effect information, the vital information, the body fluid volume information, and the serum sodium level, and input the combination to the prediction model 200 in the subsequent process.

The control unit 2 of the information processing device 1 inputs information regarding a plurality of urine outputs, the medicinal effect information, and the vital information to the prediction model 200 (S104). As described above, the control unit 2 of the information processing device 1 may further input the body fluid volume information and the serum sodium level to the prediction model 200. The information regarding a plurality of urine outputs includes the urine output at the present time, which is the urine output at the measurement time point in the most recent measurement unit time, and the value of a past urine output, which is the value of the urine output at the previous measurement time point that is one measurement unit time earlier than the most recent measurement time point. The past urine outputs may include not only the urine output at the previous measurement time point, but also the urine outputs at a plurality of measurement time points, such as the urine output at the measurement time point immediately before the previous measurement time point. That is, the information regarding a plurality of urine outputs may include urine outputs at a plurality of measurement time points after the previous measurement retroactive and a measurement unit time ago, including the urine output at the most recent (current) measurement time point. Accordingly, the information regarding a plurality of urine outputs includes the history and the transition of the urine outputs at the respective measurement time points from the past up to the present time (current measurement time), and is sorted on a time-series basis.

The control unit 2 of the information processing device 1 may convert the values of the plurality of urine outputs into urine output levels (low urine output, intermediate urine output, and excessive urine output) corresponding to the values of the urine outputs by referring to the urine output level table, for example, and set the urine output levels as the information regarding a plurality of urine outputs. In this case, the prediction model 200 is trained to output a urine output level a predetermined elapsed time ahead when a plurality of urine output levels sorted on a time-series basis is input thereto.

In the present embodiment, the information regarding a plurality of urine outputs, the medicinal effect information, and the vital information are input to the prediction model 200, but the present invention is not limited to this. The control unit 2 of the information processing device 1 may input only the information regarding a plurality of urine outputs and the medicinal effect information to the prediction model 200. In this case, the prediction model 200 is trained to output a predicted urine output a predetermined elapsed time ahead when the information regarding a plurality of urine outputs and the medicinal effect information are input thereto. Alternatively, the control unit 2 of the information processing device 1 may input only the information regarding a plurality of urine outputs to the prediction model 200. In this case, the prediction model 200 is trained to output a predicted urine output a predetermined elapsed time ahead when the information regarding a plurality of urine outputs is input thereto.

The control unit 2 of the information processing device 1 acquires information regarding a predicted urine output a predetermined time ahead, which has been estimated by the prediction model 200 (S105). On the basis of the information regarding a plurality of urine outputs, the medicinal effect information, and the vital information, which have been input, the prediction model 200 outputs information regarding a predicted urine output a predetermined elapsed time ahead, which is a predicted urine output in a future time point after a lapse of a measurement unit time (a predetermined elapsed time) since the most recent (current) measurement time point, which is the present time. The prediction model 200 may output (estimate) the urine output level corresponding to the predicted urine output as the information regarding a predicted urine output at a future time point. Like the plurality of urine outputs actually measured from the past till the present time, the urine output level corresponding to the predicted urine output may also correspond to (conform to) a urine output level defined in the urine output level table, for example. That is, the actually measured urine outputs and the predicted urine output are classified into levels according to the same definitions of urine output levels (urine output level table). In a case where the prediction model 200 outputs (estimates) the value (ml/kg/h) of the predicted urine output as the information regarding the predicted urine output at a future time point, the control unit 2 of the information processing device 1 may refer to the urine output level table and derive a urine output level corresponding to the value of the predicted urine output.

The control unit 2 of the information processing device 1 identifies the urine discharge type, on the basis of the urine output levels of the plurality of urine outputs and the predicted urine output (S106). The control unit 2 of the information processing device 1 refers to the urine output level table, and identifies the respective urine output levels of the plurality of urine outputs (actual measured urine outputs) from the past up to the present and the urine output predicted by the prediction model 200. Note that, in a case where the prediction model 200 outputs the urine output level of the predicted urine output as a prediction result, the control unit 2 of the information processing device 1 uses the urine output level. Thus, the transition of the urine output level in each measurement unit time can be derived from the past to the future.

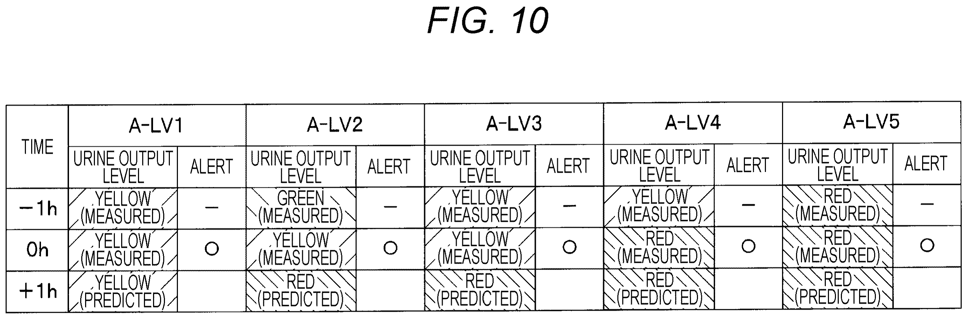

Fig. 10 is an explanatory diagram illustrating an example of transitions of urine output levels. The drawing of the present embodiment shows an example of five modes (A-LV1 to 5) with regard to the transitions of the urine output levels of urine outputs (actual measured urine outputs) at the present time (0 h) and the previous measurement time point (-1 h), and the urine output level of a predicted urine output in the future (+1 h).

In a first mode (A-LV1), the intermediate urine output (shown in yellow) continues in the previous time (past), at the present, and in the future. Accordingly, the intermediate urine output (shown in yellow) continues (is accumulated) at the three measurement time points from the past to the future (a period formed with three measurement unit times). In a case where the intermediate urine output (shown in yellow) continues a predetermined number of times or more, such as three times as above, the control unit 2 of the information processing device 1 outputs an alert.

In a second mode (A-LV2), the previous time (past) has the low urine output (shown in green), the present time has the intermediate urine output (shown in yellow), and the future has the excessive urine output (shown in red). In a case where the future urine output level is identified as the excessive urine output in this manner, the control unit 2 of the information processing device 1 outputs an alert.

In a third mode (A-LV3), the previous time (past) and the present time have the intermediate urine output (shown in yellow), and the future has the excessive urine output (shown in red). In a case where the future urine output level is identified as the excessive urine output in this manner, the control unit 2 of the information processing device 1 outputs an alert.

In a fourth mode (A-LV4), the previous time (past) has the intermediate urine output (shown in yellow), and the present time and the future have the excessive urine output (shown in red). In a case where the future urine output level is identified as the excessive urine output in this manner, the control unit 2 of the information processing device 1 outputs an alert.

In a fifth mode (A-LV5), the excessive urine output (shown in red) continues in the previous time (past), at the present, and in the future. In a case where the future urine output level is identified as the excessive urine output in this manner, the control unit 2 of the information processing device 1 outputs an alert. In a case where the intermediate urine output and the excessive urine output continue, and a case where the excessive urine output continues, the control unit 2 of the information processing device 1 may change the content of the alert to be output, depending on the degree of the continuity.

The control unit 2 of the information processing device 1 refers to the urine discharge type table, for example, and identifies the urine discharge type on the basis of the urine output levels of a plurality of urine outputs and a predicted urine output (the transition of urine output level from the past to the future). For example, in a case where the urine output level of the predicted urine output is the excessive urine output, the urine discharge type is identified as A (pattern 1) having a relatively high severity (risk). In this case, the control unit 2 of the information processing device 1 can identify the urine discharge type on the basis of the one-time item of only the urine output level of the predicted urine output.

For example, in a case where the urine output levels of a plurality of urine outputs and a predicted urine output are the intermediate urine output, which is a case where a state with the intermediate urine output continues from the past to the future, the urine discharge type is identified as B (pattern 2) having a relatively moderate severity (risk), and is classified into a polyuria warned state requiring attention though the severity (risk) is lower than that of the urine discharge type A (pattern 1). In this case, the control unit 2 of the information processing device 1 can identify the urine discharge type, on the basis of the cumulative items of the urine output levels of the plurality of urine outputs (actual measured urine outputs) from the past up to the present and the urine output level of the predicted urine output.

For example, in a case where the urine output levels of a plurality of urine outputs and a predicted urine output are the low urine output, which is a case where a state with the low urine output continues from the past to the future, the urine discharge type is identified as C (pattern 3) having a relatively low severity (risk). By referring to the urine discharge type table stored in the storage unit 3, the control unit 2 of the information processing device 1 can efficiently derive the urine discharge type, depending on the urine output level in each measurement unit time from the past to the future.

The control unit 2 of the information processing device 1 derives an intervention means including an alert, depending on the urine discharge type and the medicinal effect information (S107). By referring to the intervention means table, for example, the control unit 2 of the information processing device 1 outputs an alert corresponding to the identified urine discharge type. In a case where a urine discharge type having a relatively high severity (risk) is identified as A (pattern 1), for example, the control unit 2 of the information processing device 1 outputs an alert indicating danger to the display device 102 or the information terminal 101 held by a medical personnel. Alternatively, the control unit 2 of the information processing device 1 may output, as an alert, a voice for calling attention from a speaker or the like connected to the information processing device 1.

In a case where a urine discharge type having a relatively moderate severity (risk) is identified as B (pattern 2), for example, the control unit 2 of the information processing device 1 outputs an alert indicating a warning to the display device 102 or the information terminal 101 held by a medical personnel. In a case where a urine discharge type having a relatively low severity (risk) is identified as C (pattern 3), for example, the control unit 2 of the information processing device 1 outputs an alert indicating follow-up to the display device 102 or the information terminal 101 held by a medical personnel. Alternatively, in this case, the control unit 2 of the information processing device 1 does not necessarily output an alert. The event in which the alert is not output can suggest to the medical personnel that follow-up or the like is to be continued without any particular problem in the target patient. Further, by referring to the intervention means table, for example, the control unit 2 of the information processing device 1 derives an intervention means, depending on the combination of the identified urine discharge type and the acquired medicinal effect information. As the urine discharge type and the medicinal effect information are combined, it is possible to derive an appropriate intervention means depending on the current condition of the patient.

The control unit 2 of the information processing device 1 outputs image data including information regarding the urine discharge type and the intervention means (S108). The control unit 2 of the information processing device 1 generates image data including information regarding the transition of the identified or derived urine output level, the urine discharge type, and the intervention means, and outputs the generated image data to the display device 102 or the information terminal 101 held by a medical personnel, so that a display screen based on the image data is displayed on the display device 102 or the like.

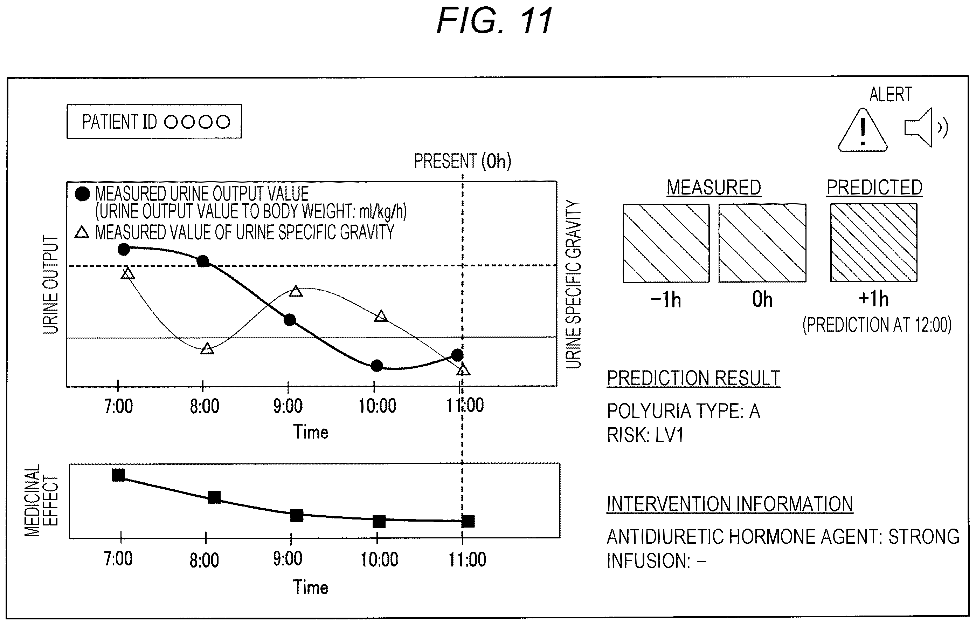

Fig. 11 is an explanatory diagram illustrating a display example (display screen) of information regarding a urine output level and the like. The control unit 2 of the information processing device 1 outputs image data including information regarding the urine discharge type, the intervention means, and the like to the display device 102 connected to the input/output I/F 4, for example. The display device 102 displays a display screen on the basis of the image data. Alternatively, the control unit 2 of the information processing device 1 outputs the image data to the information terminal 101 such as a smartphone via the communication unit 5, for example. The information terminal 101 includes a control unit, a storage unit, a communication unit, and a display unit such as a liquid crystal display like the information processing device 1, and displays a display screen on a display unit on the basis of the image data received from the information processing device 1. The display screen includes a urine output graph display area for graphically displaying the transition of measured urine outputs (urine output values to body weight: ml/kg/h) and the like, a medicinal effect graph display area for graphically displaying the transition of medicinal effects (residual effect characteristics of a medicine), and a result display area for displaying an identified urine discharge type and the like, for example.

In the urine output graph display area, a urine output graph showing the transition of urine outputs and urine specific gravities is displayed, with the vertical axis indicating the urine outputs and the urine specific gravities, the horizontal axis indicating the elapsed time. In the urine output graph, auxiliary lines indicating a first threshold (5 [ml/kg/h], for example) and a second threshold (2 [ml/kg/h], for example) to be used in identifying a urine output level may be shown. The elapsed time indicated on the horizontal axis may be represented by measurement time points formed with each measurement unit time such as one hour, for example. In this case, an auxiliary line indicating the present time, which is the most recent measurement time point (0 h), may be shown.

In the medicinal effect graph display area, a medicinal effect graph in which the vertical axis indicates medicinal effect values (the medicinal effect index) and the horizontal axis is synchronized in elapsed time with the urine output graph is displayed. From the medicinal effect graph, it can be recognized that the medicinal effect index decreases with the time elapsed since the time of administration till the present time. Since the medicinal effect graph and the urine output graph are synchronized with each other in the elapsed time indicated by the horizontal axis, the value (medicinal effect index) indicating the medicinal effect at the time point of measurement of the urine output in a measurement unit time such as one hour can be efficiently grasped, for example.

In the result display area, display objects indicating the urine output levels in the past (-1 h), at the present time (0 h), and in the future (+1 h) are displayed, for example. The display objects have a rectangular shape, and are displayed in different colors depending on the individual urine output levels (low urine output, intermediate urine output, and excessive urine output). In the present embodiment, the display object of the excessive urine output may be displayed in red, the display object of the intermediate urine output may be displayed in yellow, and the display object of the low urine output may be displayed in green.

In the result display area, a prediction result corresponding to the transition of these urine output levels from the past to a predicted future urine output is further displayed. The prediction result includes the urine discharge type derived on the basis of the transition including the urine output level of the predicted urine output, and the risk corresponding to the urine discharge type. The risk may be further subdivided on the basis of the transition pattern of the urine output level in each urine discharge type. In the result display area, intervention information derived depending on the urine discharge type is further displayed. The intervention information includes matter related to the amount of medication or necessity of infusion, for example.

According to the present embodiment, the information processing device 1 acquires information regarding a plurality of urine outputs measured from the same patient within a predetermined period, and inputs the information to the prediction model 200, to acquire, from the prediction model 200, a predicted urine output in a future time a predetermined elapsed time ahead, or, in other words, later than the time point of measurement of the urine output last measured within the predetermined period. That is, the plurality of urine outputs measured within the predetermined period include the value of the urine output (actual measured urine output value) at each predetermined measurement unit time such as one hour within the predetermined period, for example. The predicted urine output corresponds to a predicted value of a future urine output at a time point when the measurement unit time has elapsed since the time point of measurement of the urine output last measured in the predetermined period. Since the prediction model 200 has learned to output a predicted urine output a predetermined elapsed time ahead when information regarding urine outputs is input, it is possible to efficiently acquire a patient's predicted urine output in a future time, using the prediction model 200.

According to the present embodiment, the information processing device 1 accepts an input of information regarding medication for diabetes insipidus as administered to a patient, and derives medicinal effect information on the basis of the information regarding medication, to acquire the medicinal effect information, for example. The information regarding medication includes the type, amount, and time of administration of an administered medicine, for example. On the basis of the type and amount of the medicine, the information processing device 1 may derive the medicinal effect information about the administered medicine at the present time, depending on the time elapsed since the time of administration till the present time. The information processing device 1 inputs the information regarding urine outputs and the medicinal effect information to the prediction model 200, to acquire, from the prediction model 200, a predicted urine output of the patient in a future time. Since the prediction model 200 has learned to output a predicted urine output a predetermined elapsed time ahead when the information regarding urine outputs and the medicinal effect information are input, it is possible to increase the accuracy of a predicted urine output of the patient in a future time.

According to the present embodiment, the information processing device 1 acquires vital information about a patient output by various sensors such as a heart rate system attached to the patient, for example. The information processing device 1 may further acquire physical attribute information about the patient as the vital information (or in association with the vital information) from a medical information system such as an electronic medical record system that manages the physical attribute information including the sex, age, body weight, underlying disease, and the like of the patient. The body weight may be acquired from a bed equipped with a body weight measuring function (a bed with scales). The information processing device 1 inputs the information regarding urine outputs and the vital information to the prediction model 200, to acquire, from the prediction model 200, a predicted urine output of the patient in a future time. Since the prediction model 200 has learned to output a predicted urine output a predetermined elapsed time ahead when the information regarding urine outputs and the vital information are input, it is possible to increase the accuracy of a predicted urine output of the patient in a future time.

According to the present embodiment, information regarding a predicted urine output includes a urine output level defined in a plurality of stages, such as three stages (low urine output, intermediate urine output, and excessive urine output), for example, depending on the magnitude of the numerical value of the predicted urine output. Likewise, a plurality of urine outputs measured within a predetermined period from a patient are classified into urine output levels defined in a plurality of stages such as three stages (low urine output, intermediate urine output, and excessive urine output), for example, as with the predicted urine output. In classifying a predicted urine output and measured urine outputs into urine output levels, each of these urine outputs may be represented by a urine output per hour (ml/h), for example, or may be a urine output (ml/kg/h) with a body weight ratio of the patient taken into consideration (or a urine output divided by the body weight). By showing these urine outputs (a predicted urine output and measured urine outputs) at the urine output levels defined in a plurality of stages such as three stages depending on the magnitude of the numerical values of the urine outputs, the numerical values of the urine outputs can be normalized or quantized. With the use of the urine output levels thus normalized or the like, arithmetic processing for the prediction model 200 can be performed, and the accuracy of prediction performed by the prediction model 200 can be increased while the load of the arithmetic processing is reduced.

According to the present embodiment, the information processing device 1 can sort, on a time-series basis, the urine output levels identified by a plurality of urine outputs measured within a predetermined period and a predicted future urine output after the predetermined period (a plurality of measured urine outputs, and a predicted urine output). Accordingly, with a plurality of urine output levels at the present time and in the past, and a future urine output level, the transition (mode change) of these urine output levels from the past to the future can be derived. The information processing device 1 identifies the urine discharge type of the patient on the basis of the transition of the urine output levels from the past to the future thus derived, and outputs information regarding the identified urine discharge type to the display device 102 such as a display or the information terminal 101 such as a smartphone held by a medical personnel, for example. Thus, it is possible to identify the urine discharge type of the patient by taking into account not only the urine outputs from the past up to the present time but also a future urine output. Further, to derive and output an intervention means for the patient depending on the urine discharge type in view of the future state of the patient, it is expected to appropriately perform early intervention for the urine discharge type.

According to the present embodiment, urine outputs (a plurality of measured urine outputs, and a predicted urine output) are classified into urine output levels that are three stages (excessive urine output, intermediate urine output, and low urine output), for example. In the three stages, the excessive urine output as the urine output level having the largest urine output corresponds to a case where the urine output is equal to or higher than the predetermined first threshold. The low urine output as the urine output level having the lowest urine output corresponds to a case where the urine output is smaller than the second threshold, which is lower than the first threshold. The intermediate urine output as the urine output level between the excessive urine output and the low urine output corresponds to a case where the urine output is smaller than the first threshold and is equal to or larger than the second threshold.

In a case where the predicted urine output level is the excessive urine output (a level at which the urine output is equal to or larger than the first threshold), the urine discharge type of the patient is identified as a first type, and first type information indicating that the patient is identified as the first type is output. In a case where the urine output level of the predicted urine output and the urine output levels of the plurality of urine outputs are the intermediate urine output (lower than the first threshold, and equal to or higher than the second threshold), a state with the intermediate urine output has continued, the urine discharge type of the patient is identified as a second type, and second type information indicating that the patient is identified as the second type is output. In a case where the urine output level of the predicted urine output and the urine output levels of the plurality of urine outputs are the low urine output (lower than the second threshold), a state with the low urine output has continued, the urine discharge type of the patient is identified as a third type, and third type information indicating that the patient is identified as the third type is output.

As for a patient with diabetes insipidus, dehydration or the like is suspected when the urine output in a measurement unit time such as one hour is equal to or higher than the first threshold, for example. However, in a case where the urine output level of the predicted urine output corresponds to the excessive urine output (equal to or higher than the first threshold), the first type information to this effect is output, and thus, it is possible to efficiently notify a medical personnel. In a case where the urine output in a measurement unit time is lower than the first threshold, dehydration or the like is also suspected when a state with the intermediate urine output (lower than the first threshold, and equal to or higher than the second threshold) continues with the urine output levels of a plurality of urine outputs up to the present time and the urine output level of a predicted future urine output. On the other hand, in a case where the intermediate urine output (lower than the first threshold, and equal to or higher than the second threshold) continues from the past to the future including the present time, the second type information to this effect is output, and thus, it is possible to efficiently notify a medical personnel. Furthermore, in a case where the low urine output (lower than the second threshold) continues from the past to the future including the present time, the third type information to this effect is output, and thus, it is possible to efficiently notify a medical personnel that the patient is in a relatively stable state from the viewpoint of diabetes insipidus.

According to the present embodiment, the information processing device 1 generates image data including information regarding urine outputs (actual measured urine output values), information regarding a predicted urine output (a predicted urine output value), and information regarding the transition of urine output levels from the past to the future, the urine discharge type, and the intervention means, and outputs the generated image data to the display device 102 such as a display, or the information terminal 101 such as a smartphone held by a medical personnel, for example. On the screen displayed on the display device 102 according to the image data, the display objects indicating the urine output levels are displayed in different colors depending on the respective urine output levels (low urine output, intermediate urine output, and excessive urine output). For example, the display object of the excessive urine output may be displayed in red, the display object of the intermediate urine output may be displayed in yellow, and the display object of the low urine output may be displayed in green. By displaying the respective urine output levels in different colors in this manner, it is possible to efficiently cause a medical personnel to visually recognize the matter related to the transition of urine output levels of the patient.

According to the present embodiment, it is possible to efficiently generate the prediction model 200 that outputs a predicted urine output in a future time on the basis of information regarding a plurality of urine outputs measured from the same patient within a predetermined period.

### Second Embodiment

As opposed to the aspect that outputting control data for various pump devices on the basis of information regarding a plurality of urine outputs measured from the same patient within a predetermined period is not considered in the physical condition estimating system disclosed in Patent Literature 1, the disclosure in the present embodiment is to provide a computer program or the like for outputting control data for a syringe pump and an infusion pump on the basis of information regarding a plurality of urine outputs measured from the same patient within a predetermined period.

A computer program according to the present embodiment causes a computer to perform a process that includes: acquiring information regarding urine outputs measured from the same patient at a plurality of time points within a predetermined period; generating first control data for controlling a syringe pump that administers a medicine that affects urine outputs, and second control data for controlling an infusion pump that replenishes a body fluid, on the basis of the acquired information regarding the urine outputs measured at the plurality of time points; and outputting the generated first control data and second control data.

An information processing method according to the present embodiment causes a computer to perform a process that includes: acquiring information regarding urine outputs measured from the same patient at a plurality of time points within a predetermined period; generating first control data for controlling a syringe pump that administers a medicine that affects urine outputs, and second control data for controlling an infusion pump that replenishes a body fluid, on the basis of the acquired information regarding the urine outputs measured at the plurality of time points; and outputting the generated first control data and second control data.

An information processing device according to the present embodiment includes: an acquisition unit that acquires information regarding urine outputs measured from the same patient at a plurality of time points within a predetermined period; a generation unit that generates first control data for controlling a syringe pump that administers a medicine that affects urine outputs, and second control data for controlling an infusion pump that replenishes a body fluid, on the basis of the acquired information regarding the urine outputs measured at the plurality of time points; and an output unit that outputs the generated first control data and second control data.

According to the present disclosure, it is possible to provide a computer program and the like that output control data for a syringe pump and an infusion pump on the basis of information regarding a plurality of urine outputs measured from the same patient within a predetermined period.

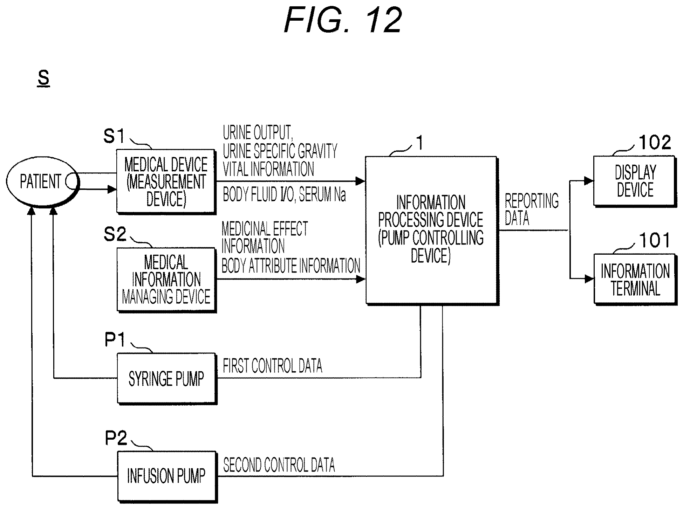

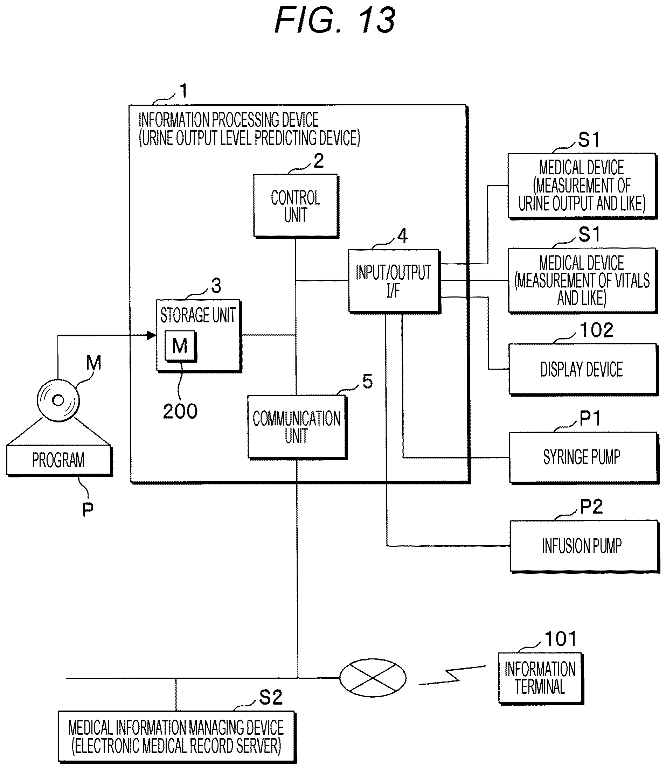

In the description below, the present disclosure is specifically described, with reference to the drawings illustrating the embodiment. Fig. 12 is a schematic diagram illustrating an outline of an information processing system S including an information processing device 1 (a pump controlling device) according to a second embodiment. Fig. 13 is a block diagram illustrating an example configuration of the information processing device 1. The information processing system S (a pump controlling system) includes the information processing device 1 (pump controlling device) as a principal device, and one or more medical devices S1 are communicably connected to the information processing device 1. The medical devices S1 include a medical device S1 for urine output measurement, a medical device S1 for urine specific gravity measurement, a medical device S1 for body fluid I/O measurement, a medical device S1 for serum sodium level measurement, and a medical device S1 for measurement of information about vital signs (hereinafter also referred to as vital information), for example.

The information processing device 1 periodically acquires, from these medical devices S1, a urine output value of the patient, a urine specific gravity, a body fluid I/O, a serum sodium level, and vital information (biological data) such as a heart rate. The information processing device 1 is further communicably connected to a medical information managing device S2 such as an electronic medical record system that is operated and managed by a medical institution, and acquires physical attribute information such as the patient's weight from the medical information managing device S2. The information processing device 1 may further acquire, from the medical information managing device S2, the type, the dosage amount, and the time point of administration of a medicine administered to the patient, or medicinal effect information. The medicine includes an antidiuretic hormone agent for central diabetes insipidus, for example.

The information processing device 1 derives an increasing/decreasing tendency (trend) in each index such as an actual measured urine output value, on the basis of various kinds of information acquired from the medical devices S1 and the medical information managing device S2. The information processing device 1 outputs the generated first control data and second control data to a syringe pump P1 (medicinal solution pump) and an infusion pump P2, on the basis of the derived increasing/decreasing tendency (trend) in each index such as an actual measured urine output value. Thus, the information processing device 1 can efficiently control the dosage amounts and administration patterns of a medicinal solution and an infusion solution for the syringe pump P1 and the infusion pump P2, depending on the increasing/decreasing tendency (trend) in each index. Further, information regarding a predicted urine output a predetermined elapsed time ahead may be derived using a prediction model 200 stored in a storage unit 3 of the information processing device 1. The prediction model 200 will be described later.

An information terminal 101 and a display device 102 are communicably connected to the information processing device 1. When outputting the first control data and the second control data generated on the basis of the increasing/decreasing tendency (trend) in each index such as an actual measured urine output value to the syringe pump P1 and the infusion pump P2, the information processing device 1 may output information regarding the dosage amounts and administration patterns of the medicinal solution and the infusion solution indicated by the first control data and the second control data, to the information terminal 101 and the display device 102 held by a medical personnel. As a result, when performing control on administration by the syringe pump P1 and the infusion pump P2, it is possible to timely notify the medical personnel beforehand of the information regarding the dosage amounts and the administration patterns, which are specific contents of the administration control.

As in the first embodiment, the information processing device 1 is a computer capable of various kinds of information processing and transmission/reception of information, and is a server device, a personal computer, or the like, for example. The server device includes not only a single server device, but also a cloud server device formed with a plurality of computers, or a virtual server device. In a case where the information processing device 1 is formed with a cloud server device, for example, like the medical devices S1, the information processing device 1 is not necessarily installed in medical facilities where the patient is present, and may be communicably connected to the medical devices S1 via an external network such as the Internet. The information processing device 1 includes a control unit 2, a communication unit 5, a storage unit 3, and an input/output I/F 4. The information processing device 1 may be included in a medical device S1 such as a urine output meter, or in the syringe pump P1 or the infusion pump P2.

The storage unit 3 stores a urine output level table, a urine discharge type table, and an intervention means table, which will be described later, in addition to a dosage amount table. The storage unit 3 further stores an entity file that constitutes the prediction model 200, which will be described later. The entity file may be formed as part of the program P (program product).

The input/output I/F 4 is a communication interface conforming to a communications standard such as RS232C or USB, for example. An input device such as a keyboard or the display device 102 such as a liquid crystal display is connected to the input/output I/F 4. Further, the medical devices S1, the syringe pump P1, and the infusion pump P2 may also be communicably connected to the input/output I/F 4.

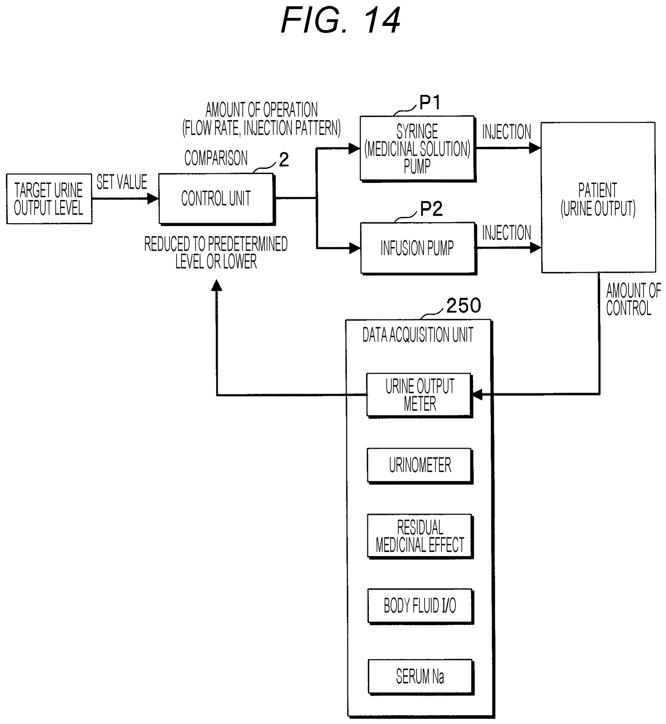

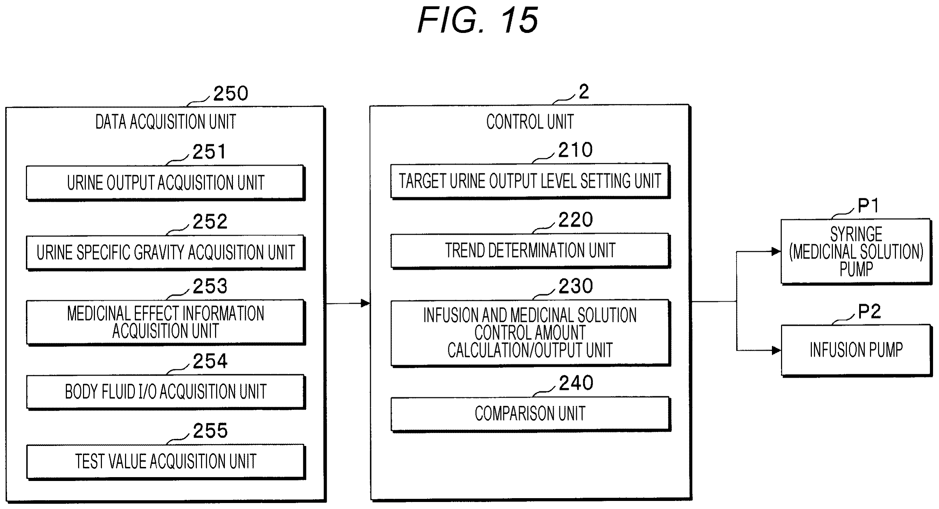

Fig. 14 is an explanatory diagram illustrating a flow of various kinds of data for the control unit 2 of the information processing device 1. Fig. 15 is a block diagram illustrating an example configuration of each functional unit formed by the control unit 2 of the information processing device 1. The control unit 2 of the information processing device 1 acquires a target urine output level as a set value, and stores the target urine output level into the storage unit 3. The control unit 2 of the information processing device 1 further derives an increasing/decreasing tendency (trend) of the value (measured value) of each index such as an actual measured urine output value acquired by a data acquisition unit 250. These respective indexes include a plurality of actual measured urine output values sorted on a time-series basis from the past up to the present time, urine specific gravity, medicinal effect information, body fluid I/O (body fluid volume information), and serum sodium level (serum Na).

On the basis of the increasing/decreasing tendency (trend) of the value of each of these indexes, the control unit 2 of the information processing device 1 refers to the dosage amount table, for example, and generates and outputs control data (the first control data and the second control data) corresponding to the amounts operation (flow rates and injection patterns of the medicinal solution and the infusion solution) for the syringe pump P1 and the infusion pump P2. The syringe pump P1 and the infusion pump P2 inject the medicinal solution and the infusion solution into the patient, depending on the control data (the first control data and the second control data) from the control unit 2 of the information processing device 1.

The control unit 2 of the information processing device 1 continuously performs a process of continuously acquiring, via the data acquisition unit 250, an actual measured urine output value that is periodically measured regarding the patient, and generating the control data (the first control data and the second control data). After the previous administration control performed on the syringe pump P1 and the infusion pump P2, the control unit 2 of the information processing device 1 compares the actual measured urine output value acquired most recently with the previously set target urine output level, and then performs a feedback process for the administration control on the syringe pump P1 and the infusion pump P2, or a process related to suspension of the administration.

In a case where the risk of diabetes insipidus is determined to be high on the basis of the urine output, the residual medicinal effect, and the urine specific gravity, the control unit 2 of the information processing device 1 may control the syringe pump P1 to perform bolus administration. In a case where the risk of diabetes insipidus is determined to be moderate on the basis of the urine output, the residual medicinal effect, and the urine specific gravity, the control unit 2 of the information processing device 1 may control the syringe pump P1 to perform continuous administration. The control unit 2 of the information processing device 1 may control the infusion pump P2 to subdivide the necessary amount of infusion, on the basis of the current value of the body fluid I/O (body fluid volume information). Further, the control unit 2 of the information processing device 1 may control the infusion pump P2, taking (adding) the body weight of the patient into consideration.

The control unit 2 of the information processing device 1 functions as functional units that perform various kinds of software processes by executing the program P stored in the storage unit 3. The functional units include a target urine output level setting unit 210, a trend determination unit 220, an infusion and medicinal solution control amount calculation/output unit 230, a comparison unit 240, and the data acquisition unit 250, for example.

The target urine output level setting unit 210 acquires an actual measured value (an actual measured urine output value) of one or more urine outputs measured before the most recent time point of urine output measurement or a target urine output level that is determined by a medical doctor or the like on the basis of the urine output level corresponding to the actual measured urine output value by receiving an input operation performed by the medical doctor or the like, for example, and stores the acquired actual measured value or target urine output level into the storage unit 3. In a case where a target urine output level is set (updated) again, the target urine output level setting unit 210 may store the updated target urine output level associated with the time point of the update, to also store a history of updates of the target urine output level.

The trend determination unit 220 derives an approximate line of each index from the past up to the present time (current time) by applying the least squares method to each index such as a plurality of actual measured urine output values acquired and sorted on a time-series basis, for example, and derives an increasing/decreasing tendency (trend) by determining the trend to be an increasing trend when the slope of the approximate line has a positive value, and determining the trend to be a decreasing trend when the slope has a negative value. The trend determination unit 220 may derive a tendency (trend) on the basis of the amount (rate) of change between past and current values. Alternatively, the trend determination unit 220 may derive a tendency (trend) on the basis of a result of regressive prediction of the tendency. Further, the trend determination unit 220 may derive an increasing/decreasing tendency (trend) of urine outputs by taking into consideration the current normalized urine output (the urine output level or the like corresponding to an actual measured urine output value) or a total urine output that has been traced back in the past by a predetermined time (a total value of urine outputs in a predetermined time in the past), in addition to a tendency (trend) of a slope, an amount of change, or the like derived from regression or the like of urine outputs or the like. The trend determination unit 220 may derive an increasing/decreasing tendency (trend) of urine outputs by taking into consideration a future urine output (a predicted urine output value or a predicted urine output level) that is predicted using the prediction model 200 described later.

The infusion and medicinal solution control amount calculation/output unit 230 generates the first control data for controlling the amount of medicinal solution administration from the syringe pump P1 and the second control data for controlling the amount of infusion solution administration from the infusion pump P2 by referring to the dosage amount table, for example, on the basis of the increasing/decreasing tendency (trend) of each index such as an actual measured urine output value as derived by the trend determination unit 220. The comparison unit 240 determines whether the actual measured urine output value measured most recently after the target urine output level was set is equal to or lower than the target urine output level that has been set by the target urine output level setting unit 210. The target urine output level has been set so that the urine output value becomes equal to or lower than a predetermined value, for example, and the comparison unit 240 compares the actual measured urine output value measured most recently with the predetermined value defined by the target urine output level, determines whether the actual measured urine output value is equal to or smaller than the predetermined value, and outputs a determination result.

The data acquisition unit 250 includes a urine output acquisition unit 251, a urine specific gravity acquisition unit 252, a medicinal effect information acquisition unit 253, a body fluid I/O acquisition unit 254, and a test value acquisition unit 255, for example. The data acquisition unit 250 acquires a value (measured value) of each index such as an actual measured urine output value, from various measurement devices included in the medical devices S1 or constituting the medical devices S1.

The urine output acquisition unit 251 may acquire the value (the total amount in the urine bag) of the urine output acquired every measurement unit time (one hour or the like, for example), and calculate a value (ml/h) of the urine output in the current measurement unit time by subtracting the value of the urine output acquired last time from the value of the urine output acquired this time. Further, the control unit 2 of the information processing device 1 may acquire physical attribute information including the body weight and the like of the patient from the medical information managing device S2, and acquire, as information regarding the urine output, the value of the urine output to the body weight obtained by dividing the value of the urine output (ml/h) in a measurement unit time by the body weight (kg) of the patient (urine output value to body weight: ml/kg/h). That is, the control unit 2 of the information processing device 1 may perform various kinds of arithmetic processing in the present embodiment, using the urine output value to body weight (ml/kg/h) as an actual measured urine output value. The urine specific gravity acquisition unit 252 acquires a urine specific gravity from a urinometer or the like among the medical devices S1 every measurement unit time (one hour or the like, for example), in synchronization with the urine output acquisition unit 251. A urine specific gravity value may be measured on the basis of a gravimetric method, a floating balance, a refractometer method, or a test paper method, for example, and be automatically acquired from a sensor device or the like, or may be acquired by receiving an input operation by an operator such as a medical personnel. Further, the amount of change in urine specific gravity in a predetermined time calculated from a plurality of urine specific gravity values actually measured and sorted on a time-series basis may be acquired as information regarding urine outputs.

The medicinal effect information acquisition unit 253 acquires, from the medical information managing device S2, for example, information regarding the type, amount, and time of administration of the medicine most recently administered to the patient, and derives (acquires) medicinal effect information at the present time by referring to the residual effect characteristics of the medicine, depending on the time elapsed since the time of administration till the present time. The medicinal effect information acquisition unit 253 may derive medicinal effect information at the present time on the basis of a function defining a medicinal effect reduction line or a medicinal effect reduction curve, depending on the time elapsed since the most recent administration of a medicine. Alternatively, the medicinal effect information acquisition unit 253 may acquire a medicine name by receiving an input operation performed by an operator such as a medical personnel from an input device such as a keyboard connected to the input/output I/F 4 of the information processing device 1, and automatically acquire the medicinal effect information on the basis of the acquired medicine name. Alternatively, the medicinal effect information acquisition unit 253 may acquire the type of the medicine, the dosage, and the patient's background such as the body weight, and calculate or estimate the medicinal effect information by taking these items into consideration. Further, the medicinal effect information acquisition unit 253 may use a value estimated by some other means.

The body fluid I/O acquisition unit 254 acquires the amount of discharge of body fluid (body fluid OUT) from the amount of intake of body fluid (body fluid IN) within a predetermined period. As for information regarding the amount of intake of body fluid (body fluid IN) and the amount of discharge of body fluid (body fluid OUT), these pieces of information may be acquired by receiving an input operation performed by an operator such as a medical personnel, from an input device such as a keyboard connected to the input/output I/F 4 of the information processing device 1. The body fluid I/O acquisition unit 254 may acquire only the amount of intake of body fluid (body fluid IN). In this case, the amount of discharge of body fluid (body fluid OUT) may be an actual measured urine output value. The body fluid I/O acquisition unit 254 calculates the current body fluid volume information (body fluid I/O), which is body fluid balance, by subtracting the amount of discharge of body fluid (body fluid OUT) from the amount of intake of body fluid (body fluid IN) within the predetermined period. Note that the amount of intake of body fluid (body fluid IN) may be calculated as a total value of the amount of infusion, the amount of drinking water, the amount of medicinal solution, and the like, for example. The amount of discharge of body fluid (body fluid OUT) may be calculated as a total value of the urine output, the stool output, the amount of drainage, the amount of insensible water loss, and the like, for example. In this case, the control unit 2 (biological data acquisition unit) may also function as the body fluid I/O acquisition unit 254. Note that the body fluid volume information may be defined (formed) only by information regarding the amount of intake of body fluid (body fluid IN). That is, in the case of a patient with diabetes insipidus, the amount of discharge of body fluid (body fluid OUT) is substantially equal to the urine output, and accordingly, only the amount of intake of body fluid (body fluid IN) may be used as the body fluid volume information. By grasping the body fluid balance of the patient at the present time (current time) in greater detail, it is possible to estimate an optimal amount of infusion that reduces the burden on the patient and reduces the risk of dehydration. By outputting the estimate of the amount of infusion, it is possible to present useful information to the medical personnel.

The test value acquisition unit 255 acquires the serum sodium level (serum Na level) from a medical device S1 every measurement unit time (one hour or the like, for example). Alternatively, the test value acquisition unit 255 may acquire information regarding the serum sodium level (serum Na level) and the antidiuretic hormone level by receiving an input operation performed by an operator such as a medical personnel, from an input device such as a keyboard connected to the input/output I/F 4 of the information processing device 1.

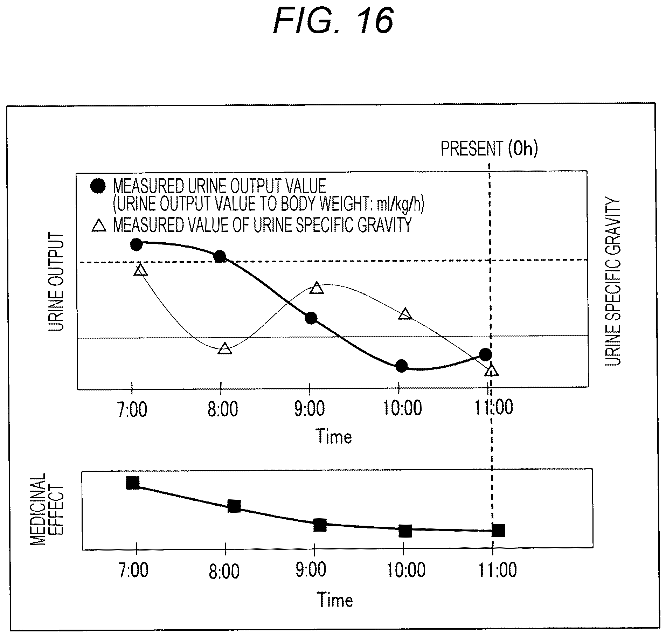

Fig. 16 is an explanatory diagram illustrating an example of transition of actual measured urine output values and the like. In the drawing according to the present embodiment, a urine output graph and a medicinal effect graph are shown. The urine output graph shows the transition of urine outputs and urine specific gravities, with the vertical axis indicating the urine outputs and the urine specific gravities, the horizontal axis indicating the elapsed time. In the urine output graph, auxiliary lines indicating a first threshold (5 [ml/Kg/h], for example) and a second threshold (2 [ml/Kg/h], for example) to be used in identifying a urine output level may be shown. The elapsed time indicated on the horizontal axis may be represented by measurement time points formed with each measurement unit time such as one hour, for example. In this case, an auxiliary line indicating the present time, which is the most recent measurement time point (0 h), may be shown. The urine output (actual measured urine output value) and the urine specific gravity measured every measurement unit time in this manner are associated with the measurement time point and are stored in the storage unit 3.

In the medicinal effect graph, the vertical axis indicates medicinal effect values (the medicinal effect index), and the horizontal axis is synchronized in elapsed time with the urine output graph. Information regarding the medicinal effect graph may be stored in the storage unit 3, depending on the type of medicine administered through the syringe pump P1. Thus, the control unit 2 of the information processing device 1 can recognize, from the medicinal effect graph, that the medicinal effect index decreases with the time elapsed since the time of administration till the present time. Further, since the medicinal effect graph and the urine output graph are synchronized with each other in the time indicated by the horizontal axis, the value (medicinal effect index) indicating the medicinal effect at the time point of measurement of the urine output in a measurement unit time such as one hour can be efficiently grasped, for example.

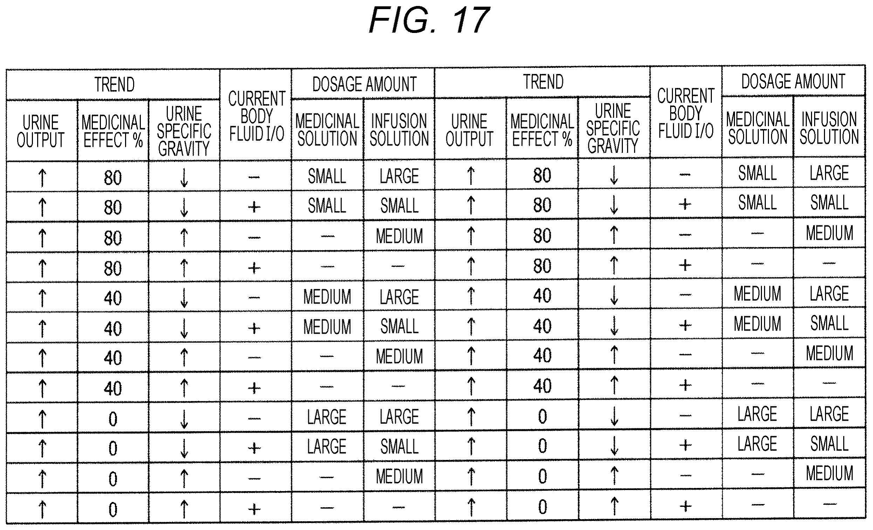

Fig. 17 is an explanatory diagram illustrating an example of the dosage amount table. The dosage amount table is stored in the storage unit 3. The control unit 2 of the information processing device 1 generates the first control data and the second control data by referring to the dosage amount table stored in the storage unit 3. The management items (fields) in the dosage amount table include urine output, medicinal effect %, urine specific gravity, current body fluid I/O, medicinal solution, and infusion solution, for example.

In a urine output management item, a tendency (trend) indicating an increase or decrease in the urine output (actual measured urine output value) in a predetermined period is stored. In the present embodiment, an upward arrow indicates an increasing trend, and a downward arrow indicates a decreasing trend. In a management item of medicinal effect %, the residual effect corresponding to the time elapsed since the time of administration is stored. The residual effect may be represented by a value obtained by setting a medicinal effect index at the time of administration to 100% and decreasing the medicinal effect index with the elapsed time. In a urine specific gravity management item, a tendency (trend) indicating an increase or decrease in the urine specific gravity in a predetermined period is stored. In the present embodiment, an upward arrow indicates an increasing trend, and a downward arrow indicates a decreasing trend.

In a current body fluid I/O management item, an increase or decrease in the body fluid volume in a predetermined period, which is a body fluid balance, is stored. In a case where the amount of intake of body fluid (body fluid IN) within a fixed period is larger than the amount of discharge of body fluid (body fluid OUT), the body fluid balance to be stored is positive (+). In a case where the amount of intake of body fluid is smaller than the amount of discharge of body fluid, the body fluid balance to be stored is negative (-).

In a medicinal solution management item, the medicinal solution dosage amount corresponding to the values of the management items of the urine output, the medicinal effect %, the urine specific gravity, and the current body fluid I/O stored in the same record is stored. The medicinal solution dosage amount is equivalent to (corresponds to) the first control data for controlling the syringe pump P1. In the present embodiment, as an example, "small" indicates a small dosage amount of the medicinal solution, "large" indicates a large dosage amount of the medicinal solution, and "-" indicates that the dosage amount of the medicinal solution is none.

In an infusion solution management item, the infusion solution dosage amount corresponding to the values of the management items of the urine output, the medicinal effect %, the urine specific gravity, and the current body fluid I/O stored in the same record is stored. The infusion solution dosage amount is equivalent to (corresponds to) the second control data for controlling the infusion pump P2. In the present embodiment, as an example, "small" indicates a small dosage amount of the infusion solution, "large" indicates a large dosage amount of the infusion solution, and "-" indicates that the dosage amount of the infusion solution is none.

The dosage amount table may further include management items that store tendencies (trends) of the serum sodium level and the antidiuretic hormone level. For example, in the case of a patient with a small body weight such as an infant, even when the amount of urine discharge is small over several hours, body fluid is positively balanced and retained by infusion in preparation for polyuria, and, at this point of time, the serum sodium level exhibits a lowering (decreasing) trend. Therefore, the syringe pump P1 and the infusion pump P2 are controlled, with the tendency (trend) of the serum sodium level being taken into consideration. Thus, it is possible to estimate an optimal amount of infusion or the like with a smaller burden on the patient and a lower risk of dehydration. Further, symptoms of diabetes insipidus vary from patient to patient due to the influence of operation of neurosurgery on a tumor such as a pituitary adenoma, and there are cases where antidiuretic hormone is not semi-permanently secreted, or secretion of antidiuretic hormone is recovered after a lapse of a certain period of time after surgery, and the degrees of symptoms vary. For example, in the case of polyuria during a period in which secretion of antidiuretic hormone is recovered, it is considered that the body fluid has a positive balance because of infusion or the like. In such a case, it is conceivable to reduce the amount of medicine and the amount of infusion. Therefore, the syringe pump P1 and the infusion pump P2 are controlled, with the tendency (trend) of the antidiuretic hormone level being taken into consideration. Thus, an optimal amount of medicine, an optimal amount of infusion, and the like can be estimated.

In the dosage amount table, values that can be taken as values for urine outputs, medicinal effects %, urine specific gravities, current body fluid I/Os, serum sodium levels, medicinal solutions, and infusion solutions, which are management items, are comprehensively placed. Accordingly, the control unit 2 of the information processing device 1 can appropriately control the syringe pump P1 and the infusion pump P2 by referring to the dosage amount table. The value or content of each of the items defined or managed in the dosage amount table is not necessarily set in a fixed manner, but may be variably set depending on an input or a change operation performed by an operator of the information processing device 1, such as a medical personnel. That is, the control unit 2 of the information processing device 1 may receive an input or a change operation performed by an operator such as a medical personnel, and change or update the dosage amount table, depending on the received operation content. For example, as for a medical act of determining the dosage amounts, administration patterns, and the like to be adopted by the syringe pump P1 and the infusion pump P2, even in a case where there is a difference in the determination criteria for the medical act depending on each medical institution, changes in the settings or definitions in the dosage amount table can be allowed, and thus, availability of the information processing system S (a pump controlling system) can be increased.

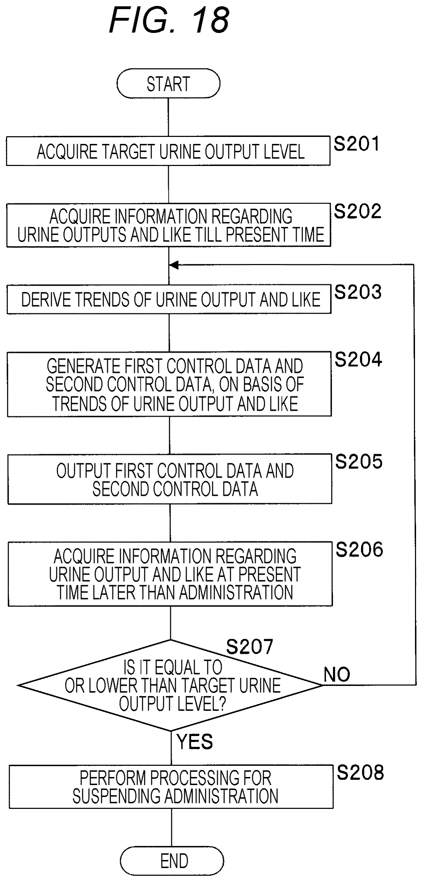

Fig. 18 is a flowchart illustrating an example of processing procedures (actual measured urine output values in the past and till the present time) to be carried out by the control unit 2 of the information processing device 1. The control unit 2 of the information processing device 1 accepts an operator's operation using a keyboard or the like connected to the input/output I/F 4, for example, and performs the following process on the basis of the accepted operation.

The control unit 2 of the information processing device 1 acquires a target urine output level (S201). The control unit 2 of the information processing device 1 acquires a target urine output level determined by a medical doctor or the like on the basis of one or more actual measured values of urine outputs (actual measured urine output values) or urine output levels corresponding to the actual measured urine output values by receiving an input operation performed by the medical doctor or the like, for example. The control unit 2 of the information processing device 1 associates the acquired target urine output level with the time point of the acquisition of the target urine output level (the time point of the input operation performed by the medical doctor or the like), and stores the acquired target urine output level into the storage unit 3. The target urine output level stored in the storage unit 3 in this manner is not stored as a fixed or uniform value, and thereafter, when an input operation by a medical doctor or the like is further performed, the target urine output level may be updated by further receiving the input operation. The control unit 2 of the information processing device 1 can also store a history of updating of the target urine output level, by storing the acquired target urine output level associated with the acquisition time point.

The control unit 2 of the information processing device 1 acquires information regarding urine outputs and the like obtained up to the present time (S202). The control unit 2 of the information processing device 1 further acquires urine specific gravity, body fluid volume information, and information regarding serum sodium level, for example, in addition to the actual measured urine output values from the past up to the present. The control unit 2 of the information processing device 1 acquires information regarding a plurality of urine outputs of the same patient within a predetermined period, by periodically or regularly acquiring a urine output value output from a medical device S1 that performs urine output measurement, for example. The medical device S1 for urine output measurement measures the value of a urine output stored in a urine bag of the same patient every measurement unit time such as one hour, for example, and outputs the measured value (the value of the urine output [ml]) to the information processing device 1.

The control unit 2 of the information processing device 1 may acquire the value (the total amount in the urine bag) of the urine output acquired every measurement unit time (one hour or the like, for example), and calculate a value (ml/h) of the urine output in the current measurement unit time by subtracting the value of the urine output acquired last time from the value of the urine output acquired this time. Further, the control unit 2 of the information processing device 1 may acquire physical attribute information including the body weight and the like of the patient from the medical information managing device S2, and acquire, as information regarding the urine output, the value of the urine output to the body weight obtained by dividing the value of the urine output (ml/h) in a measurement unit time by the body weight (kg) of the patient (urine output value to body weight: ml/kg/h). Further, the control unit 2 of the information processing device 1 may acquire, together with the urine output, urine specific gravity output from the medical device S1 that performs urine output measurement, and acquire the acquired urine specific gravity as the information regarding the urine output.

The control unit 2 of the information processing device 1 may further acquire body fluid volume information or serum sodium level, or both pieces of information. In this case, the body fluid volume information may include only the amount of intake of body fluid (body fluid IN), or may include the amount of intake of body fluid (body fluid IN) and the amount of discharge of body fluid (body fluid OUT). The medicinal effect information, the vital information, the body fluid volume information, and the serum sodium level may be data at the present time, or may be formed with a plurality of pieces of data sorted on a time-series basis. The control unit 2 of the information processing device 1 does not necessarily acquire all the values (indexes) of the urine specific gravity, the body fluid volume information, and the serum sodium level, and may acquire any one or more of these indexes.

The control unit 2 further acquires medicinal effect information about the medicine for diabetes insipidus that is currently administered to the patient. The control unit 2 of the information processing device 1 acquires, from the medical information managing device S2, information regarding the type, amount, and time of administration of the medicine most recently administered to the patient, and derives (acquires) medicinal effect information at the present time by referring to the residual effect characteristics of the medicine, depending on the time elapsed since the time of administration till the present time. In the medicinal effect information, a medicinal effect index at the time of administration is set to 100%, for example, and the medicinal effect index may be lowered over the lapse of time.

The control unit 2 may further acquire vital information about the patient. The control unit 2 of the information processing device 1 acquires vital information that is output from a medical device S1 that measures vital information (biological data) regarding heart rate, blood pressure, and the like, for example. The control unit 2 of the information processing device 1 may acquire various kinds of data such as the value of a urine output, the medicinal effect information, and the vital information (biological data), by receiving an input operation by an operator such as a medical personnel from an input device such as a keyboard connected to the input/output I/F 4. The accuracy of the first control data and the second control data can be increased by taking the vital information into consideration when these sets of data are generated. The control unit 2 of the information processing device 1 associates the acquired values (various kinds of data) of the respective indexes with the acquisition time points, and stores these values into the storage unit 3.

The control unit 2 of the information processing device 1 derives trends of urine outputs and the like (S203). The control unit 2 of the information processing device 1 derives tendencies (trends) indicating an increase or decrease in urine outputs and the like, on the basis of the acquired information regarding a plurality of urine outputs and the like sorted on a time-series basis. The control unit 2 of the information processing device 1 derives a urine output tendency (trend) indicating an increasing/decreasing trend of actual measured urine output values, using the least squares method on the plurality of acquired actual measured urine output values sorted on a time-series basis, for example. The plurality of actual measured urine output values sorted on a time-series basis may be actual measured urine output values at two measurement time points, which are the most recent measurement time point at the present time and the immediately preceding measurement time point, or may be actual measured urine output values at three or more measurement time points including the measurement time point at the present time and the two or more preceding measurement time points. The control unit 2 of the information processing device 1 derives an approximate line based on a plurality of actual measured urine output values from the past up to the present (current time) by applying the least squares method to the plurality of actual measured urine output values at the present time and one or more past measurement time points, and derives an increasing/decreasing tendency (trend) by determining the trend to be an increasing trend when the slope of the approximate line has a positive value, and determining the trend to be a decreasing trend when the slope has a negative value.

The control unit 2 of the information processing device 1 also derives an increasing/decreasing tendency (trend) for a plurality of urine specific gravities sorted on a time-series basis, as in the case with actual measured urine output values. The control unit 2 of the information processing device 1 calculates the current body fluid volume information (body fluid I/O), which is the body fluid balance, by subtracting the amount of discharge of body fluid (body fluid OUT) from the amount of intake of body fluid (body fluid IN) within a predetermined period. In a case where the amount of intake of body fluid (body fluid IN) within the predetermined period is larger than the amount of discharge of body fluid (body fluid OUT), the body fluid balance is positive (+). In a case where the amount of intake of body fluid is smaller than the amount of discharge of body fluid, the body fluid balance is negative (-). The control unit 2 of the information processing device 1 also derives an increasing/decreasing tendency (trend) for a plurality of serum sodium levels (serum Na) sorted on a time-series basis, as in the case with actual measured urine output values. In this manner, the control unit 2 of the information processing device 1 derives increasing/decreasing tendencies (trends) in each index such as acquired urine outputs sorted on a time-series basis.

The control unit 2 of the information processing device 1 generates the first control data and the second control data, on the basis of the tendencies of urine outputs and the like (S204). The control unit 2 of the information processing device 1 generates the first control data and the second control data by referring to the dosage amount table, for example, on the basis of the plurality of actual measured urine output values sorted on a time-series basis, the increasing/decreasing tendency (trend) of the actual measured urine output values, the medicinal effect information (medicinal effect %) at the present time, and the body fluid balance (body fluid I/O) within the predetermined period. The dosage amount table stores values indicating the dosage amounts of the medicinal solution and the infusion solution depending on the increasing/decreasing tendency (trend) in each of the indexes including urine outputs and the like, and the control unit 2 of the information processing device 1 generates the first control data (the dosage amount of the medicinal solution) and the second control data (the dosage amount of the infusion solution) corresponding to those dosage amounts. By generating the first control data (the dosage amount of the medicinal solution) and the second control data (the dosage amount of the infusion solution) corresponding to the dosage amounts in this manner, it is possible to adjust the conditions for drive control on the syringe pump P1 and the infusion pump P2.

The control unit 2 of the information processing device 1 outputs the generated first control data and second control data (S205). The control unit 2 of the information processing device 1 outputs the generated first control data to the syringe pump P1, and outputs the second control data to the infusion pump P2. As a result, the syringe pump P1 and the infusion pump P2 may start administration according to these pieces of control data (the first control data and the second control data). Alternatively, the syringe pump P1 and the infusion pump P2 may start administration of the medicinal solution and the infusion solution by receiving an administration start operation performed by a medical doctor or the like after the medical doctor or the like checks the conditions such as the dosage amounts of the medicinal solution and the infusion solution with the first control data and the second control data.

The control unit 2 of the information processing device 1 acquires information regarding the urine output and the like at the present time, which is later than the time of administration (S206). After a predetermined measurement unit time such as one hour has elapsed, for example, the control unit 2 of the information processing device 1 acquires the information regarding the urine output and the like at the present time, which is later than the immediately preceding administration. The control unit 2 of the information processing device 1 periodically acquires an actual measured urine output value, a urine specific gravity, and medicinal effect information every measurement unit time such as one hour, for example. Likewise, the control unit 2 of the information processing device 1 periodically acquires body fluid volume information and a serum sodium level. Further, the control unit 2 of the information processing device 1 acquires these indexes in the same manner as in the processing in S202, associates the indexes with the acquisition time points, and stores the indexes into the storage unit 3. By associating the respective indexes such as the periodically acquired actual measured urine output values with the acquisition time points and storing these indexes into the storage unit 3, it is possible to save and manage the history of temporal change in each of the indexes such as the actual measured urine output values.

The control unit 2 of the information processing device 1 determines whether the urine output is equal to or lower than the target urine output level (S207). The control unit 2 of the information processing device 1 determines whether the actual measured urine output value at the present time later than the administration, or the actual measured urine output value most recently measured, is equal to or lower than the target urine output level. The target urine output level is set so that each urine output value is equal to or smaller than a predetermined value, for example. The control unit 2 of the information processing device 1 determines whether the actual measured urine output value most recently measured is equal to or smaller than the predetermined value defined as the target urine output level. The control unit 2 of the information processing device 1 determines the actual measured urine output value to be equal to or lower than the target urine output level when the actual measured urine output value is equal to or smaller than the predetermined value, but determines the actual measured urine output value to be higher than the target urine output level when the actual measured urine output value is greater than the predetermined value.

The control unit 2 of the information processing device 1 may perform a process related to comparison between an actual measured urine output value and the target urine output level in a longer period of time than a measurement unit time for periodically acquiring an actual measured urine output value. That is, in a case where the measurement unit time for an actual measured urine output value is one hour, for example, the control unit 2 of the information processing device 1 may set the processing unit time related to the comparison with the target urine output level to three hours or the like, which is a longer time (cycle) than the measurement unit time for an actual measured urine output value.

If the actual measured urine output value is higher than the target urine output level (S207: NO), the control unit 2 of the information processing device 1 performs a loop process to again perform the process starting from S203. By again performing the process from S203 to S207, the control unit 2 of the information processing device 1 again derives (re-derives) an increasing/decreasing tendency (trend) in each of the indexes such as actual measured urine output values, on the basis of the information regarding the urine output and the like at the present time later than administration, and the information regarding the urine outputs and the like acquired before at measurement time points in the past. After that, the control unit 2 of the information processing device 1 again generates (regenerates) control data (the first control data for the medicine, and the second control data for the infusion solution) corresponding to the dosage amounts, by referring to the dosage amount table, on the basis of the re-derived increasing/decreasing tendency (trend) in each of the indexes.

The control unit 2 of the information processing device 1 may correct the regenerated first control data and second control data, on the basis of the difference between the actual measured urine output value at the present (most recent) time later than the administration and the target urine output level. The control unit 2 of the information processing device 1 calculates the difference value between the predetermined value defined as the target urine output level and the actual measured urine output value at the present (most recent) time later than the administration, by subtracting these values. The control unit 2 of the information processing device 1 may derive a correction factor on the basis of the calculated difference value, and correct the first control data and the second control data, using the correction factor.

The control unit 2 of the information processing device 1 may identify the most recent urine output level corresponding to the actual measured urine output value at the present (most recent) time later than the administration, and determine whether the identified most recent urine output level and the target urine output level are the same urine output levels. In a case where the most recent urine output level and the target urine output level are not the same urine output levels, which is a case where the most recent urine output level and the target urine output level are different, and there is a difference (difference value), the first control data and the second control data may be corrected on the basis of the difference (difference value). Accordingly, a feedback process depending on the difference between the most recent urine output (urine output value), which is the urine output at the present time, and the target urine output level can be performed on the first control data and the second control data generated on the basis of the tendency (trend) in each of the indexes such as a plurality of urine outputs sorted on a time-series basis, and thus, the first control data and the second control data corrected to have higher accuracy can be output.

If the actual measured urine output value is equal to or lower than the target urine output level (S207: YES), the control unit 2 of the information processing device 1 performs a process for suspending the administration (S208). In a case where the actual measured urine output value at the present (most recent) time later than the administration is equal to or lower than the target urine output level, it can be said that the urine discharge pattern of the patient is in a normal state. The control unit 2 of the information processing device 1 performs a process for suspending the administration being performed by the syringe pump P1 and the infusion pump P2, by outputting the first control data and the second control data indicating administration suspension toward the syringe pump P1 and the infusion pump P2, or by stopping the output of the first control data and the second control data.

In the present embodiment, the control unit 2 of the information processing device 1 determines whether to continue the administration of the medicine or the infusion solution, or to suspend the administration (complete the administration), depending on the comparison with the target urine output level, but the present invention is not limited to this. For example, in every measurement unit time that is one hour, a process for continuing or completing (stopping) the administration of the medicine or the infusion solution may be performed on the basis of the increasing/decreasing tendency (trend) in each of the indexes such as urine outputs from the past up to the present time. That is, an increasing/decreasing tendency (trend) in each of the indexes such as urine outputs derived in each measurement unit time may be derived, the dosage amounts (large, small, none) of the medicine and the infusion solution may be determined by referring to the dosage amount table, for example, and the control data (the first control data for the medicine, and the second control data for the infusion solution) corresponding to the determined dosage amounts may be generated and output. In the dosage amount table, values (control parameters) indicating that the dosage amounts of the medicine and the infusion solution are none (administration is suspended) are also stored (defined), and thus, even in a case where only the dosage amount table is used, continuation or suspension (completion) of the administration of the medicine and the infusion solution can be efficiently controlled.

According to the present embodiment, the information processing device 1 acquires, from a medical device S1 for urine output measurement, for example, information regarding a plurality of urine outputs from the same patient that are measured within a predetermined period and are sorted on a time-series basis. Alternatively, the information processing device 1 may acquire information regarding a plurality of urine outputs sorted on a time-series basis, by receiving an input operation performed by an operator (a medical personnel or the like) of the information processing device 1 from an input device such as a keyboard connected to the input/output I/F 4 of the information processing device 1. The plurality of urine outputs measured within the predetermined period include the value of the urine output (actual measured urine output value) in each predetermined measurement unit time such as one hour within the predetermined period, for example. On the basis of the acquired information regarding a plurality of urine outputs sorted on a time-series basis, the information processing device 1 generates the first control data for controlling the syringe pump P1 that administers the antidiuretic, and the second control data for controlling the infusion pump P2 that replenishes body fluid. The information processing device 1 outputs the generated first control data to the syringe pump P1, and outputs the second control data to the infusion pump P2. In accordance with the first control data and second control data output by the information processing device 1, administration of the medicinal solution and the infusion solution from the syringe pump P1 and the infusion pump P2 to the patient is started. Alternatively, the syringe pump P1 and the infusion pump P2 may start administration by receiving an administration start operation performed by a medical doctor or the like after the medical doctor or the like checks the conditions such as the dosage amounts of the medicinal solution and the infusion solution with the first control data and the second control data. With this arrangement, it is possible to appropriately support the medical personnel and the like in suppressing and stabilizing the polyuria symptoms by controlling the balance between the water content of the infusion solution and the amount of the medicinal solution, depending on the information regarding a plurality of urine outputs that are measured from the same patient within a predetermined period and are sorted on a time-series basis.

According to the present embodiment, the information processing device 1 may acquire at least one or more pieces of information among medicinal effect information about the medicine for diabetes insipidus administered to the patient, vital information about the patient, body fluid volume information about the patient, and serum sodium level, in addition to the information regarding a plurality of urine outputs that are measured from the same patient within a predetermined period and are sorted on a time-series basis. The information processing device 1 generates and outputs the first control data and the second control data, on the basis of a combination of the one or more pieces of information and the information regarding urine outputs. The combination of the one or more pieces of information includes the modes among all combinations of the medicinal effect information about the medicine for diabetes insipidus administered to the patient, the vital information about the patient, the body fluid volume information about the patient, and the serum sodium level. As described above, the first control data and the second control data are generated on the basis of each of the indexes including one or more pieces of information among the medicinal effect information about the medicine for diabetes insipidus administered to the patient, the vital information about the patient, the body fluid volume information about the patient, and the serum sodium level, in addition to the information regarding the urine outputs. Thus, the accuracy of generation of the first control data and the second control data can be increased.

According to the present embodiment, the information regarding a plurality of urine outputs sorted on a time-series basis includes a urine output trend derived on the basis of a plurality of actual measured urine output values sorted on a time-series basis. In this case, the information processing device 1 may derive a urine output tendency (trend) indicating an increasing/decreasing trend of the actual measured urine output values in the period (measurement period) corresponding to the time series, on the basis of the acquired information (actual measured urine output values) regarding a plurality of urine outputs sorted on a time-series basis. The information processing device 1 may derive a urine output tendency (trend) indicating an increasing/decreasing trend of actual measured urine output values, using the least squares method on the plurality of acquired actual measured urine output values sorted on a time-series basis, for example. The information processing device 1 may generate the first control data and the second control data by referring to the dosage amount table stored in the storage unit 3, on the basis of the derived urine output tendency (trend). As described above, by observing the pattern of the tendencies (trends) in the indexes such as actual measured urine output values, and simultaneously controlling the medicinal solution and the infusion solution and injecting the medicinal solution and the infusion solution into the patient at an early stage before the occurrence or deterioration of a polyuria symptom, it is possible to support or promote the decrease in the risk of dehydration of the patient, and the stabilization of the polyuria symptom at an early stage. The tendencies (trends) in the indexes are not limited to the tendency (urine output tendency) of actual measured urine output values, and may include trends (a urine specific gravity trend, a body fluid volume information trend, and a serum sodium level trend) of the respective values indicating increasing/decreasing trends in urine specific gravity, body fluid volume information, and serum sodium level, for example. As the first control data and the second control data are generated on the basis of the tendencies (trends) in a plurality of indexes in this manner, the accuracy of these pieces of data can be further increased.

According to the present embodiment, the information regarding urine outputs includes a total value of urine outputs and the urine specific gravity in a predetermined measurement unit time that is one hour, for example. The information processing device 1 acquires, from the medical device S1 for urine output measurement, for example, information regarding a plurality of urine outputs from the same patient that are measured within a predetermined period and are sorted on a time-series basis. In this case, the medical device S1 for urine output measurement may include a urine output meter and a urinometer. As the information processing device 1 generates the first control data and the second control data on the basis of each of the indexes including the urine outputs and the urine specific gravity included in the information regarding urine outputs, the accuracy of generation of the first control data and the second control data can be increased.

According to the present embodiment, the information processing device 1 acquires information regarding the most recent urine output (the most recent urine output actual measurement value or the most recent urine output level) included in the information regarding a plurality of urine outputs sorted on a time-series basis, and further acquires a target urine output level determined earlier than the time point of measurement of the most recent urine output. The target urine output level may be determined by a medical doctor or the like, on the basis of one or more actual measured values of urine outputs (actual measured urine output values) measured earlier than the measurement time point of the most recent urine output or the urine output level corresponding to the actual measured urine output value(s). The information processing device 1 acquires the target urine output level by receiving an input operation performed by a medical doctor or the like, and stores the acquired target urine output level into the storage unit 3. In performing this process, when acquiring the most recent urine output level or the like corresponding to the most recent urine output, the information processing device 1 acquires the already set target urine output level by referring to the storage unit 3. The information processing device 1 determines a difference between the acquired most recent urine output level or the like and the target urine output level, and whether the most recent urine output level or the like and the target urine output level are the same urine output levels. In a case they are different, which is a case where there is a difference (difference value) between the most recent urine output level or the like and the target urine output level, the information processing device 1 corrects the first control data and the second control data on the basis of the difference (difference value). Accordingly, a feedback process depending on the difference between the most recent urine output (urine output value), which is the urine output at the present time, and the target urine output level can be performed on the first control data and the second control data generated on the basis of the information regarding a plurality of urine outputs sorted on a time-series basis and the like, and thus, the first control data and the second control data corrected to have higher accuracy can be output.

### Third Embodiment

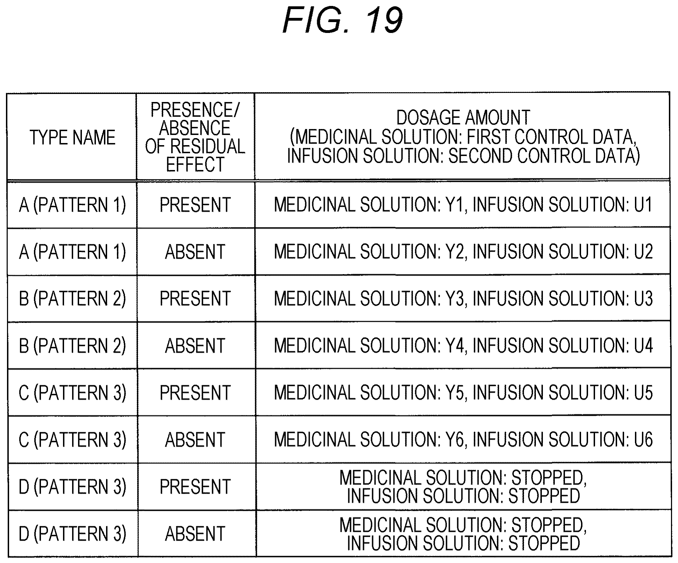

Fig. 19 is an explanatory diagram illustrating an example of an intervention means table. The management items (fields) in the intervention means table include type name, presence or absence of residual effect, and dosage amount, for example. In a type name management item, a type name defined in a urine discharge type table is stored. With this arrangement, the intervention means table and the urine discharge type table are normalized by the type name management items. In a management item of the presence or absence of a residual effect, a value (presence/absence) indicating the presence or absence of a residual effect is stored under the respective type names (A to D, for example) defined by the urine discharge types.

In a dosage amount management item, information regarding the dosage amounts and the administration patterns of the medicinal solution and the infusion solution corresponding to the combination of the type name and the presence or absence (presence/absence) of a residual effect stored in the same record is stored. The information regarding the dosage amounts and administration patterns of the medicinal solution and the infusion solution is equivalent to (corresponds to) control data (first control data and second control data) for a syringe pump P1 and an infusion pump P2, and is intended as an intervention means for the patient. As the combinations are defined, a control unit 2 of an information processing device 1 can efficiently derive an intervention means (the dosage amounts and the administration patterns to be adopted by the syringe pump P1 and the infusion pump P2) corresponding to the combination of the identified urine discharge type and the acquired medicinal effect information.

In the present embodiment, the classification of the urine output levels and the urine discharge types is an example, and the present invention is not limited to this. The urine output levels may be further subdivided and defined depending on the urine output values. The urine discharge types may be further subdivided and defined depending on modes of transitions (changes) of a plurality of urine output levels that are continuous in time series. Further, the value or content of each of the items defined or managed in the urine output level table, the urine discharge type table, and the intervention means table is not necessarily set in a fixed manner, but may be variably set depending on an input or a change operation performed by an operator of the information processing device 1, such as a medical personnel. That is, the control unit 2 of the information processing device 1 may receive an input or a change operation performed by an operator such as a medical personnel, and change or update the urine output level table, the urine discharge type table, or the intervention means table, depending on the received operation content. For example, as for the definition of a urine discharge type or the like, even in a case where there is a difference in the criteria of the definition at each medical institution, it is possible to allow changes in the settings or definitions in each table in this manner, and increase availability of an information processing system S (a pump controlling system).

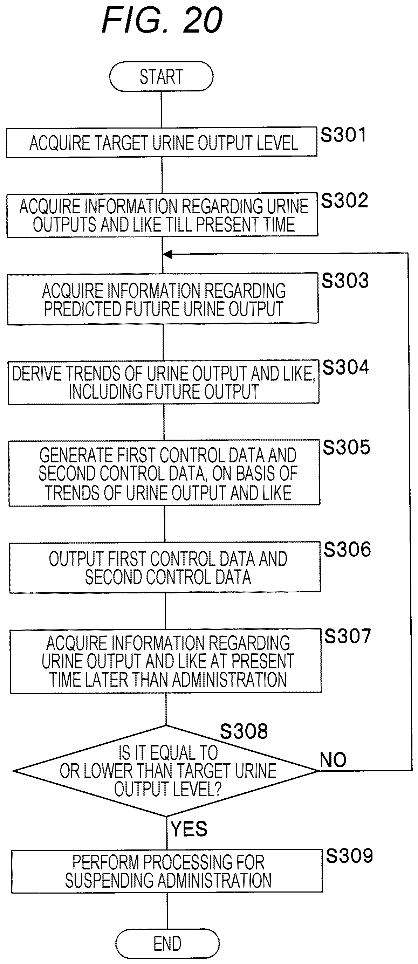

Fig. 20 is a flowchart illustrating an example of processing procedures (actual measured urine output values and a predicted urine output value) to be carried out by the control unit 2 of the information processing device 1 according to a third embodiment. The control unit 2 of the information processing device 1 accepts an operator's operation using a keyboard or the like connected to the input/output I/F 4, for example, and performs the following process on the basis of the accepted operation.

The control unit 2 of the information processing device 1 acquires a target urine output level (S301). The control unit 2 of the information processing device 1 acquires information regarding urine outputs and the like obtained up to the present time (S302). The control unit 2 of the information processing device 1 performs the process from S301 to S302 in the same manner as the process from S201 to S202 of the second embodiment.

The control unit 2 of the information processing device 1 acquires information regarding a predicted urine output in a future time (S303). The control unit 2 of the information processing device 1 acquires the information regarding a predicted urine output in a future time, by inputting information regarding a plurality of urine outputs up to the present time, medicinal effect information, and the like to a prediction model 200. Alternatively, the control unit 2 of the information processing device 1 may input, to the prediction model 200, information regarding urine specific gravity, body fluid volume information, and serum sodium level, in addition to the information regarding a plurality of urine outputs obtained up to the present time (a plurality of actual measured urine output values sorted on a time-series basis). The information regarding a predicted urine output in a future time output by the prediction model 200 may be a predicted urine output value, or may be a predicted urine output level corresponding to the predicted urine output value.

The control unit 2 of the information processing device 1 derives trends in urine outputs including future outputs and the like (S304). The control unit 2 of the information processing device 1 derives a trend of urine outputs including future outputs, as in S203 of the second embodiment, including information (a predicted urine output value or a predicted urine output level) regarding a predicted urine output in a future time acquired from the prediction model 200. The control unit 2 of the information processing device 1 may derive a urine output tendency (trend) indicating an increasing/decreasing trend of urine output values as in the second embodiment, on the basis of actual measured urine output values and a predicted urine output value as in the second embodiment. Alternatively, the control unit 2 of the information processing device 1 may classify actual measured urine output values and a predicted urine output value into corresponding urine output levels (the most recent urine output level, a predicted urine output level, and the like), and derive a change/transition (temporal change) of these urine output levels from the past to the future or from the present time to the future, and a trend of urine outputs including a future output or the like.

The control unit 2 of the information processing device 1 generates the first control data and the second control data, on the basis of the trend of urine outputs or the like (S305). When generating the first control data and the second control data on the basis of the trend of urine outputs or the like, the control unit 2 of the information processing device 1 may generate these sets of control data by referring to a dosage amount table as in the second embodiment. Alternatively, when deriving a change/transition (temporal change) of urine output levels (the most recent urine output level, a predicted urine output level, and the like) as a trend of urine outputs or the like including a future output, the control unit 2 of the information processing device 1 may generate the first control data and the second control data by referring to the intervention means table, for example, depending on a urine discharge type defined by the change in urine output level.

The control unit 2 of the information processing device 1 outputs the generated first control data and second control data (S306). The control unit 2 of the information processing device 1 acquires information regarding the urine output and the like at the present time, which is later than the time of administration (S307). The control unit 2 of the information processing device 1 determines whether the urine output is equal to or lower than the target urine output level (S308). The control unit 2 of the information processing device 1 performs a process for suspending the administration (S309). The control unit 2 of the information processing device 1 performs the process from S306 to S309 in the same manner as the process from S205 to S208 of the second embodiment. As described above, appropriate control on the dosage amounts to be adopted by the syringe pump P1 and the infusion pump P2 can be continuously performed on the basis of a urine output tendency (trend) including a predicted future urine output value estimated with the prediction model 200, in addition to the actual measured urine output values obtained up to the present time.

According to the present embodiment, the information processing device 1 acquires information regarding a plurality of urine outputs measured from the same patient within a predetermined period, and inputs the information to the prediction model 200, to acquire, from the prediction model 200, a predicted urine output in a future time a predetermined elapsed time ahead, or, in other words, later than the time point of measurement of the urine output last measured within the predetermined period. That is, the plurality of urine outputs measured within the predetermined period include the value of the urine output (actual measured urine output value) at each predetermined measurement unit time such as one hour within the predetermined period, for example. The predicted urine output corresponds to a predicted value of a future urine output at a time point when the measurement unit time has elapsed since the time point of measurement of the urine output last measured in the predetermined period. Since the prediction model 200 has learned to output a predicted urine output a predetermined elapsed time ahead when information regarding urine outputs is input, it is possible to efficiently acquire a patient's predicted urine output in a future time, using the prediction model 200. As the information regarding a predicted urine output is used in addition to actual measured urine output values in this manner, the accuracy of the first control data and the second control data to be generated can be increased.

According to the present embodiment, information regarding a predicted urine output includes a urine output level defined in a plurality of stages, such as three stages (low urine output, intermediate urine output, and excessive urine output), for example, depending on the magnitude of the numerical value of the predicted urine output. Likewise, a plurality of urine outputs measured within a predetermined period from a patient are classified into urine output levels defined in a plurality of stages such as three stages (low urine output, intermediate urine output, and excessive urine output), for example, as with the predicted urine output. In classifying a predicted urine output and measured urine outputs into urine output levels, each of these urine outputs may be represented by a urine output per hour (ml/h), for example, or may be a urine output (ml/kg/h) with a body weight ratio of the patient taken into consideration (or a urine output divided by the body weight). By showing these urine outputs (a predicted urine output and measured urine outputs) at the urine output levels defined in a plurality of stages such as three stages depending on the magnitude of the numerical values of the urine outputs, the numerical values of the urine outputs can be normalized or quantized. With the use of the urine output levels thus normalized or the like, arithmetic processing for the prediction model 200 can be performed, and the accuracy of prediction performed by the prediction model 200 can be increased while the load of the arithmetic processing is reduced.

According to the present embodiment, the information processing device 1 identifies a urine discharge type indicating the degree of polyuria risk of the patient on the basis of the transition of a plurality of urine output levels sorted on a time-series basis including at least the most recent urine output level and a predicted urine output level, and thus, can efficiently identify the urine discharge type of the patient. After that, the information processing device 1 generates and outputs the first control data and the second control data, depending on the degree of the polyuria risk indicated by the urine discharge type. As a result, it is possible to generate appropriate first control data and second control data corresponding to the degree of polyuria risk indicated by the urine discharge type.

It should be understood that the embodiments disclosed herein are examples in all respects and are not restrictive. The scope of the present invention is presented not by the above meaning but by the claims, and is intended to include all changes within the meaning and scope equivalent to the claims.

The claims disclosed in the Claims can be combined with each other, regardless of citation forms. The Claims may recite a multiple dependent claim that depends from multiple claims. A multiple dependent claim dependent on a multiple dependent claim may be recited. Even if any multiple dependent claim dependent on a multiple dependent claim is not recited, this does not limit recitation of a multiple dependent claim dependent on a multiple dependent claim.

### Reference Signs List

S information processing system (urine output level predicting system)
S1 medical device
S2 medical information managing device
1 information processing device (urine output level predicting device)
2 control unit
21 biological data acquisition unit
22 medication information acquisition unit
23 urine output level setting unit
24 learning data generation unit
25 prediction model construction unit
26 urine output level prediction unit
27 alert output unit
271 actual urine output level acquisition unit
272 one-time alert determination unit
273 cumulative alert determination unit
274 output unit
28 intervention means display unit
281 alert information acquisition unit
282 display unit
200 prediction model
210 target urine output level setting unit
220 trend determination unit
230 infusion and medicinal solution control amount
calculation/output unit
240 comparison unit
250 data acquisition unit
251 urine output acquisition unit
252 urine specific gravity acquisition unit
253 medicinal effect information acquisition unit
254 body fluid I/O acquisition unit
255 test value acquisition unit
3 storage unit
M recording medium
P program (program product)
4 input/output I/F
5 communication unit
101 information terminal
102 display device

## Claims

1. A program for causing a computer to perform a process including:
acquiring information regarding urine outputs measured from a same patient at a plurality of time points within a predetermined period;
inputting the acquired information regarding the urine outputs measured at the plurality of time points to a prediction model that has learned to output information regarding a predicted urine output a predetermined elapsed time ahead when the information regarding the urine outputs measured at the plurality of time points is input;
acquiring the information regarding the predicted urine output the predetermined elapsed time ahead, from the prediction model; and
outputting the acquired information regarding the predicted urine output.

2. The program according to claim 1, wherein
medicinal effect information about a medicine that has been administered to the patient and affects a urine output is acquired,
the prediction model has learned to output the information regarding the predicted urine output the predetermined elapsed time ahead when the information regarding the urine outputs and the medicinal effect information are input, and
the acquired information regarding the urine outputs measured at the plurality of time points and the acquired medicinal effect information are input to the prediction model.

3. The program according to claim 1, wherein
information regarding vital signs of the patient is acquired,
the prediction model has learned to output the information regarding the predicted urine output the predetermined elapsed time ahead when the information regarding the urine outputs measured at the plurality of time points and the information regarding vital signs are input, and
the acquired information regarding the urine outputs measured at the plurality of time points and the acquired information regarding vital signs are input to the prediction model.

4. The program according to claim 1, wherein
body fluid volume information about the patient is acquired,
the prediction model has learned to output the information regarding the predicted urine output the predetermined elapsed time ahead when the information regarding the urine outputs measured at the plurality of time points and the body fluid volume information are input, and
the acquired information regarding the urine outputs measured at the plurality of time points and the acquired body fluid volume information are input to the prediction model.

5. The program according to claim 1, wherein
a serum sodium level of the patient is acquired,
the prediction model has learned to output the information regarding the predicted urine output the predetermined elapsed time ahead when the information regarding the urine outputs measured at the plurality of time points and the serum sodium level are input, and
the acquired information regarding the urine outputs measured at the plurality of time points and the acquired serum sodium level are input to the prediction model.

6. The program according to claim 1, wherein
the information regarding the predicted urine output is a predicted urine output level corresponding to a urine output level defined in a plurality of stages determined beforehand on a basis of a value of a urine output.

7. The program according to claim 6, wherein
the information regarding the urine outputs measured at the plurality of time points includes a plurality of actual measured urine output values sorted on a time-series basis.

8. The program according to claim 7, wherein
a most recent urine output level corresponding to at least a most recent urine output in the information regarding the urine outputs measured at the plurality of time points is acquired,
a urine discharge type indicating a degree of a polyuria risk in the patient is identified on a basis of transition of a plurality of urine output levels sorted on a time-series basis including at least the most recent urine output level and the predicted urine output level, and,
when the degree of the polyuria risk indicated by the identified urine discharge type is not lower than a predetermined degree, information indicating that the degree of the polyuria risk is not lower than the predetermined degree is output.

9. The program according to claim 8, wherein
an intervention means for the patient is derived depending on the identified urine discharge type, and
information regarding the derived intervention means is output.

10. The program according to claim 8, wherein
the urine output levels include:
a high level corresponding to a urine output that is not lower than a predetermined first threshold; and
an intermediate level corresponding to a urine output that is lower than the first threshold and is not lower than a second threshold lower than the first threshold,
the urine discharge types include:
a first risk type in which the predicted urine output level is the high level; and
a second risk type in which each of the urine output levels sorted on a time-series basis is the intermediate level,
when the identified urine discharge type is the first risk type, first type information is output, and,
when the identified urine discharge type is the second risk type, second type information is output.

11. The program according to claim 10, wherein
the urine discharge types further include a third risk type in which the most recent urine output level is the high level, and the predicted urine output level is the intermediate level, and,
when the identified urine discharge type is the third risk type, third type information is output.

12. The program according to claim 11, wherein
the urine output levels further include a low level corresponding to a urine output lower than the second threshold, and
the urine discharge type indicating that the polyuria risk in the patient is low or none is a type in which the predicted urine output level is the low level, or the most recent urine output level is the low level while the predicted urine output level is not the high level.

13. The program according to claim 12, wherein,
when the identified urine discharge type is the urine discharge type indicating that the polyuria risk in the patient is low or none, fourth type information is output.

14. The program according to claim 8, wherein
a past urine output level corresponding to a urine output in a time series immediately before a most recent time in the information regarding the urine outputs measured at the plurality of time points is acquired, and
the urine discharge type of the patient is identified on a basis of the transition of the plurality of urine output levels sorted on a time-series basis further including the past urine output level.

15. The program according to claim 8, wherein
image data including information regarding the transition of the plurality of urine output levels sorted on a time-series basis is generated, and the generated image data is output.

16. The program according to claim 15, wherein
image data including information regarding the urine discharge type and an intervention means is generated, and
the generated image data is output.

17. The program according to claim 16, wherein
a display object that is included in the image data and indicates the urine output level of the predicted urine output is displayed in different colors corresponding to predefined stages.

18. The program according to claim 1, wherein
the information regarding the urine outputs measured at the plurality of time points includes a plurality of actual measured urine output values sorted on a time-series basis, and the information regarding the predicted urine output is a predicted urine output value.

19. The program according to claim 18, wherein,
when the predicted urine output value is a urine output not lower than a predetermined first threshold, first type information is output, and,
when at least a most recent actual measured urine output value among the predicted urine output value and the plurality of actual measured urine output values sorted on a time-series basis is lower than the first threshold and is not lower than a second threshold that is lower than the first threshold value, second type information is output.

20. The program according to claim 1, wherein
the information regarding the urine outputs measured at the plurality of time points includes a plurality of urine specific gravity values that are actually measured and are sorted on a time-series basis.

21. The program according to claim 20, wherein
the information regarding the urine outputs measured at the plurality of time points includes an amount of change in urine specific gravity, the amount of change being calculated from the plurality of urine specific gravity values.

22. An information processing method for causing a computer to perform a process including:
acquiring information regarding urine outputs measured from a same patient at a plurality of time points within a predetermined period;
inputting the acquired information regarding the urine outputs measured at the plurality of time points to a prediction model that has learned to output information regarding a predicted urine output a predetermined elapsed time ahead when the information regarding the urine outputs measured at the plurality of time points is input;
acquiring the information regarding the predicted urine output the predetermined elapsed time ahead, from the prediction model; and
outputting the acquired information regarding the predicted urine output.

23. An information processing device comprising:
an acquisition unit that acquires information regarding urine outputs measured from a same patient at a plurality of time points within a predetermined period;
an input unit that inputs the acquired information regarding the urine outputs measured at the plurality of time points to a prediction model that has learned to output information regarding a predicted urine output a predetermined elapsed time ahead when the information regarding the urine outputs measured at the plurality of time points is input; and
an output unit that outputs the information regarding the predicted urine output the predetermined elapsed time ahead, the information regarding the predicted urine output being acquired from the prediction model.

24. A model generation method for causing a computer to perform a process including:
acquiring training data in which a plurality of urine outputs measured at a plurality of time points within a predetermined period is associated with information regarding a future urine output; and,
on a basis of the training data, generating a prediction model that has learned to output information regarding a predicted future urine output when the urine outputs measured at the plurality of time points are input.

25. A program for causing a computer to perform a process including:
acquiring information regarding urine outputs measured from a same patient at a plurality of time points within a predetermined period;
generating first control data for controlling a syringe pump that administers a medicine that affects an urine output, and second control data for controlling an infusion pump that replenishes body fluid, on a basis of the acquired information regarding the urine outputs measured at the plurality of time points; and
outputting the generated first control data and second control data.

26. The program according to claim 25, wherein
medicinal effect information about the medicine that has been administered to the patient and affects the urine output is acquired; and
the first control data and the second control data are generated on a basis of the acquired information regarding the urine outputs measured at the plurality of time points and the acquired medicinal effect information.

27. The program according to claim 25, wherein
information regarding vital signs of the patient is acquired; and
the first control data and the second control data are generated on a basis of the acquired information regarding the urine outputs measured at the plurality of time points and the acquired information regarding vital signs.

28. The program according to claim 25, wherein
body fluid volume information about the patient is acquired; and
the first control data and the second control data are generated on a basis of the acquired information regarding the urine outputs measured at the plurality of time points and the acquired body fluid volume information.

29. The program according to claim 25, wherein
a serum sodium level of the patient is acquired; and
the first control data and the second control data are generated on a basis of the acquired information regarding the urine outputs measured at the plurality of time points and the acquired serum sodium level.

30. The program according to claim 25, wherein
the information regarding the urine outputs measured at the plurality of time points includes a plurality of actual measured urine output values that are actually measured and are sorted on a time-series basis.

31. The program according to claim 30, wherein
the information regarding the urine outputs measured at the plurality of time points includes a urine output trend that is derived on a basis of the plurality of actual measured urine output values that are actually measured and are sorted on a time-series basis.

32. The program according to claim 30, wherein
the information regarding the urine outputs measured at the plurality of time points includes a total value of urine outputs in a predetermined measurement unit time.

33. The program according to claim 25, wherein
the information regarding the urine outputs measured at the plurality of time points includes a plurality of actual measured urine specific gravities that are actually measured and are sorted on a time-series basis.

34. The program according to claim 25, wherein
information regarding a most recent urine output included in the information regarding the urine outputs measured at the plurality of time points is acquired,
a target urine output level determined before a measurement time point of the most recent urine output is acquired,
the generated first control data and second control data are corrected depending on a result of comparison between the target urine output level and the information regarding the most recent urine output; and
the corrected first control data and second control data are output.

35. The program according to claim 25, wherein
the information regarding the urine outputs measured at the plurality of time points is input to a prediction model that has learned to output information regarding a predicted urine output a predetermined elapsed time ahead when the information regarding the urine outputs measured at the plurality of time points is input,
the information regarding the predicted urine output the predetermined elapsed time ahead is acquired from the prediction model, and
the first control data and the second control data are generated on a basis of the acquired information regarding the predicted urine output and the acquired information regarding the urine outputs measured at the plurality of time points.

36. The program according to claim 35, wherein
the information regarding the predicted urine output is a predicted urine output level corresponding to a urine output level defined in a plurality of stages determined beforehand on a basis of a value of an urine output.

37. The program according to claim 36, wherein
a most recent urine output level corresponding to at least a most recent urine output is acquired from the information regarding the urine outputs measured at the plurality of time points,
a urine discharge type indicating a degree of a polyuria risk in the patient is identified on a basis of transition of a plurality of urine output levels sorted on a time-series basis including at least the most recent urine output level and the predicted urine output level, and,
when the degree of the polyuria risk indicated by the identified urine discharge type is not lower than a predetermined degree, the first control data and the second control data generated depending on the degree of the polyuria risk are output.

38. An information processing method for causing a computer to perform a process including:
acquiring information regarding urine outputs measured from a same patient at a plurality of time points within a predetermined period;
generating first control data for controlling a syringe pump that administers a medicine that affects an urine output, and second control data for controlling an infusion pump that replenishes body fluid, on a basis of the acquired information regarding the urine outputs measured at the plurality of time points; and
outputting the generated first control data and second control data.

39. An information processing device comprising:
an acquisition unit that acquires information regarding urine outputs measured from a same patient at a plurality of time points within a predetermined period;
a generation unit that generates first control data for controlling a syringe pump that administers a medicine that affects a urine output, and second control data for controlling an infusion pump that replenishes body fluid, on a basis of the acquired information regarding the urine outputs measured at the plurality of time points; and
an output unit that outputs the generated first control data and second control data.
